# EUROPÄISCHE PATENTSCHRIFT

(11) **EP 2 822 538 B1**
(45) Veröffentlichungstag und Bekanntmachung des Hinweises auf die Patenterteilung: **02.03.2016**
(21) Anmeldenummer: 13707656.8
(22) Anmeldetag: 07.03.2013
(51) Int. Cl.: A61K 9/16, A61K 9/28, A61K 47/32

(54) **HERSTELLUNG VON PHARMAZEUTISCHEN PROTEKTIVEN ÜBERZÜGEN MIT GUTER RESISTENZ IM NEUTRALEN MILIEU**
PRODUCTION OF PHARMACEUTICAL PROTECTIVE OVERALLS WITH GOOD RESISTANCE IN A NEUTRAL ENVIRONMENT
FABRICATION DE REVÊTEMENTS DE PROTECTION PHARMACEUTIQUES AYANT UNE BONNE RÉSISTANCE EN MILIEU NEUTRE

(30) Priorität: 09.03.2012 EP 12158728
(43) Veröffentlichungstag der Anmeldung: 14.01.2015
(73) Patentinhaber: BASF SE, 67056 Ludwigshafen (DE)
(72) Erfinder: KOLTER, Karl, 67117Limburgerhof (DE); GEBERT, Silke, 67269 Grünstadt (DE); MÜLLER, Michael, Klemens, 67454 Haßloch (DE); ANGEL, Maximilian, 67105 Schifferstadt (DE)
(86) Internationale Anmeldenummer: PCT/EP2013/054549
(87) Internationale Veröffentlichungsnummer: WO 2013/131986

(56) Entgegenhaltungen:
- WO-A1-2009/016258
- WO-A2-2010/139654
- DE-A1- 10 239 999

## Beschreibung

Die vorliegende Erfindung betrifft pharmazeutische protektive Überzüge mit guter Resistenz im neutralen Milieu, wobei pharmazeutische Dosierungsformen zwecks Geschmacksmaskierung oder zum Schutz gegen Feuchtigkeit mit einem Filmüberzug auf Basis eines teilneutralisiert vorliegenden kationischen Copolymers, welches mittels radikalischer Emulsionspolymerisation eines N,N-Diethylaminoethylmethacrylat enthaltenden Monomergemischs erhalten wird, versehen werden. Die vorliegende Erfindung betrifft weiterhin die Herstellung entsprechender Überzüge und Verwendung von entsprechenden Überzugsmitteln für die Herstellung von protektiven Überzügen mit guter Resistenz im neutralen Milieu.

Bei der Herstellung von pharmazeutischen Dosierungsformen von unangenehm schmeckenden Wirkstoffen besteht das grundsätzliche Problem, die Dosierungsformen mit Überzügen zu versehen, die einerseits im neutralen Milieu der Mundhöhle eine gute Geschmacksmaskierung erlauben, andererseits aber auch nicht zu große Schichtdicken aufweisen. Auch bei der Herstellung von überzogenen Arzneiformen, die zum Zwecke des Schutzes von feuchtigkeitsempfindlichen Wirkstoffen mit protektiven Überzügen versehen werden, stellt sich dieses Problem.

Zu dicke Überzüge sind nicht nur ästhetisch unbefriedigend, weil sie eher rau als schön glatt sind, sondern auch wirtschaftlich unbefriedigend, weil zur Erzeugung größerer Schichtdicken mehr Überzugsmittel benötigt wird und außerdem die Prozesszeiten länger sind. Hinzu kommt, dass zu dicke Überzüge dann im sauren Milieu des Magens, wo sie den Wirkstoff möglichst schnell freisetzen sollten, eine unerwünschte langsamere Freisetzung des Wirkstoffs verursachen. Das Ziel bei der Entwicklung von Überzugsmitteln wird daher immer sein, möglichst dünne Filmüberzüge zu erzeugen, die allerdings die funktionalen Ansprüche, wie im vorliegenden Fall der Geschmacksmaskierung, erfüllen müssen. Wünschenswerte Auftragsmengen liegen daher üblicherweise im Bereich von 1 bis 20 mg/cm².

Die Überzüge müssen auch für eine ausreichende Zeit hinsichtlich der Geschmacksmaskierung stabil sein. Gerade bei Dosierungsformen, die im Mund schnell zerfallen (sogenannte "Oral Dispersible Tablets"), in denen mit einem geschmacksmaskierten Überzug versehene Wirkstoffkristalle oder Wirkstoffmikropellets in einer oral schnell zerfallenden Matrix eingebettet sind, kommt es häufig vor, dass sich die überzogenen Wirkstoffkristalle oder Mikropellets für eine Weile in Zahnzwischenräumen verhaken. Weist der Überzug dann keine ausreichende Dichtigkeit gegen Zerfall im neutralen Milieu der Mundhöhle auf, so kann es zu unangenehmen Geschmacksempfindungen dadurch kommen. Die Anforderung an geeignete Überzüge ist deshalb eine ausreichende Resistenz gegen Freisetzung des Wirkstoffs bei neutralen pH-Werten von mindestens einer Stunde, vorzugsweise sogar von zwei Stunden.

Eine weitere Anforderung ist die gute Redispergierbarkeit der filmbildenden Polymere, die die Basis der verwendeten Überzugsmittel bilden, in Wasser. Die zur Geschmacksmaskierung verwendeten filmbildenden Polymere sind üblicherweise pH-abhängig lösliche Polymere, die im neutralen Milieu wasserunlöslich sind, sich im sauren Milieu des Magens jedoch meist schnell auflösen, um eine schnelle Freisetzung des Wirkstoffs zu ermöglichen.

Die DE-AS 2512238 lehrt zur Bereitstellung von Bindemitteln für Arzneimittelüberzüge mit geringem Restmonomerengehalt die Verwendung eines durch Sprühtrocknen einer Polymerdispersion erhaltenen Pulvers zur Herstellung von Überzugslösungen für diese Arzneiformen. Bezüglich der zur Sprühtrocknung eingesetzten Dispersionen wird auf die DE 1090381, DE 1219175 und DE 2135073 Bezug genommen.

Die DE 3049179 A1 ist eine Zusatzanmeldung zur DE 2512238 und betrifft die Verwendung eines durch Sprühtrocknen nach der Lehre des letztgenannten Dokuments gewonnenen Pulvers in Form einer wässrigen Suspension, die zusätzlich ein Weichmachungsmittel enthält, zur Herstellung von Überzügen durch Thermogelierung.

Die WO 00/05307 beschäftigt sich mit der Bereitstellung von Überzugs- und Bindemitteln für Arzneiformen, die (Meth)acrylat-Copolymere enthalten, die Monomerreste mit tertiären Aminogruppen aufweisen, wobei eine einfache trockene oder wässrige Weiterverarbeitung möglich sein soll.

Die WO 02/067906 betrifft Überzugs- und Bindemittel mit verbesserter Wasserdampfdurchlässigkeit gegenüber den in der WO 00/05307 beschriebenen. Dabei erfolgt die Herstellung der Überzugs- und Bindemittel mit einer Mischung, die (a) ein Copolymer aus C₁-C₄-Estern der (Meth)acrylsäure und weitere (Meth)acrylat-Monomere mit funktionellen tertiären Ammoniumgruppen in Pulverform mit einer mittleren Teilchengröße von 1 bis 40 µm, (b) einen Emulgator mit einem HLB-Wert von mindestens 14 und (c) eine C₁₂-C₁₈-Monocarbonsäure oder eine C₁₂-C₁₈-Hydroxylverbindung enthält.

Die WO 2004/019918 beschreibt Überzugs- und Bindemittel, die bezüglich ihrer Zusammensetzung den in der WO 00/05307 und WO 02/067906 beschriebenen entsprechen.

In der EP88951 A2 ist ein Verfahren zum Überziehen von Arzneimitteln mittels eines in Wasser dispergierten Überzugsmittels auf Basis von Emulsionspolymerisaten beschrieben, wobei die Überzugsmittel partiell in Salzform vorliegen können. Die Überzugsmittel können auch aus redispergierten Pulvern erhalten werden.

In der WO 97/42255 ist die Sprühtrocknung von in wässriger Lösung redispergierbaren Polymerpulvern, enthaltend freie säuren- oder basen-gruppentragende Copolymere, beschrieben, wobei vor der Sprühtrocknung die pH-Werte der Dispersionen mit Hilfe eine Puffersystems eingestellt werden müssen.

In der EP 262326 A2 ist ein Verfahren zur Herstellung eines redispergierbaren Kunststoffpulvers beschrieben.

Aus der WO 2009/016258 ist die Herstellung der wässrigen Polymerdispersionen kationischer Polymere auf Basis von N,N-Diethylaminoethylmethacrylat wie sie erfindungsgemäß zum Einsatz kommen und deren Verwendung zum Überziehen von Arzneistoffen bekannt. Zwar wird beschrieben, dass die kationischen Polymere teilneutralisiert werden können, wobei ganz allgemein ein Reihe von anorganischen und organischen Säuren als für die Teilneutralisierung geeignet aufgelistet werden.

Bei den bisher bekannten Überzugsmitteln, die in teilneutralisierter Form vorliegen, besteht jedoch das Problem, dass die damit überzogenen Dosierungsformen eine unbefriedigende Resistenz gegen vorzeitigen Zerfall im neutralen Milieu der Mundhöhle aufweisen. Auch die Redispergierbarkeit ist häufig nicht zufriedenstellend.

Aufgabe der vorliegenden Erfindung war es, Überzugsmittel zur Verfügung zu stellen, die der pharmazeutischen Dosierungsform eine gute Resistenz gegen vorzeitige Wirkstofffreisetzung verleihen und gleichzeitig auch eine gute Redispergierbarkeit in Wasser aufweisen.

Demgemäß wurden pharmazeutische filmbildenden Überzügen erhalten aus Überzugsmitteln auf Basis von Copolymeren aus N,N-Diethylaminoethylmethacrylat und Methytmethacrylat im Gewichtsverhältnis der Monomeren von 35:65 bis 55:45, wobei die Copolymeren au 2 bis 15 mol % mit C₃-C₁₀-Dicarbonsäuren teilneutralisiert vorliegen, gefunden.

Gemäß einer bevorzugten Ausführungsform werden die Überzugsmittel in Auftragsmengen von 1 bis 20 mg/cm² aufgetragen und die Überzüge weisen bei einer Auftragsmenge von 4 mg/cm² eine Resistenz gegen Freisetzung des Wirkstoffs im wässrigen Milieu bei pH-Wert 6,8 von mindestens 80 % nach 30 min auf.

Weiterhin wurde ein Verfahren zur Herstellung von Überzugsmitteln für Überzüge von mindestens einen pharmazeutischen Wirkstoff enthaltende Dosierungsformen mit einer Resistenz gegen vorzeitige Freisetzung des Wirkstoffs gefunden, wobei die Überzugsmittel als filmbildende Polymere Copolymere aus N,N-Diethylaminoethylmethacrylat und Methylmethacrylat im Gewichtsverhältnis der Monomeren von 35:65 bis 55:45 enthalten, welches dadurch gekennzeichnet ist, dass die Copolymere im Überzugsmittel au 2 bis 15 mol% mit C₃-C₁₀-Dicarbonsäuren teilneutralisiert werden.

Gemäß einer bevorzugten Ausführungsweise werden die Überzugsmittel in Pulverform erhalten und vor dem Auftragen auf die Dosierungsform in Wasser redipergiert.

Weiterhin wurde die Verwendung von filmbildenden Überzugsmitteln für pharmazeutische Dosierungsformen auf Basis von Copolymeren aus N,N-Diethylaminoethylmethacrylat und Methylmethacrylat im Gewichtsverhältnis der Mono-meren von 35:65 bis 55:45, wobei die Copolymeren au 2 bis 15-mol-% mit C₃-C₁₀-Dicarbonsäuren teil-neutralisiert vorliegen, zum Schutz von wirkstoffhaltigen pharmazeutischen Dosierungs-formen gegen vorzeitige Wirkstofffreisetzung im wässrigen Milieu bei pH 6,8, gefunden.

Die Resistenz gegen vorzeitige Freisetzung des pharmazeutischen Wirkstoffs im wässrigen Milieu bei pH-Werten im Bereich von 6,8 wird mit Hilfe des sogenannten "Paddle-Modells" bestimmt. Diese Messmethode ist in der USP beschrieben. Dabei werden die überzogenen Dosierungsformen in Phosphatpuffer bei pH 6,8 +/- 0.05 mit Hilfe einer sogenannten "Paddle-Apparatur"(Apparatur 2) nach USP getestet. Die Herstellung des Phosphatpuffers erfolgt durch Lösen von 34,025 g Kalium-dihydrogenphosphat in Wasser in einem geeichten 5 I-Messkolben, Zugabe von 112 ml 1 molarer NaOH und Auffüllen bis zur Eichmarke des 5 I-Messkolbens. Erfindungsgemäße Überzüge erfüllen das Kriterium, dass sie bei einer Auftragsmenge von 4 mg/cm² eine Resistenz gegen Freisetzung des Wirkstoffs im wässrigen Milieu bei pH-Wert 6,8 von mindestens 80 % nach 30 min aufweisen.

Die Messung erfolgt unter Atmosphärendruck bei 25 °C. Die Freisetzung des Wirkstoffs erfolgt durch photometrische Bestimmung. Die Freisetzung wird im Abstand von 30 min. bestimmt.

Eine Resistenz von 80 % nach 30 min bedeutet erfindungsgemäß, dass nach 30 min nicht mehr als 20 % des Wirkstoffs freigesetzt sind. Vorzugsweise beträgt die Resistenz nach 30 min 100 %, was bedeutet, dass in diesem Zeitraum keine nachweisbaren Mengen (kleiner 2 %) an Wirkstoff aus den Dosierungsformen freigesetzt werden. Nach 60 min soll die Resistenz bei pH 6,8 mindestens 60 % liegen.

Erfindungsgemäß geeignete Dicarbonsäuren weisen eine Kettenlänge von 3 bis 10 Kohlenstoffatomen auf. Geeignete Dicarbonsäuren sind insbesondere unverzweigte Dicarbonsäuren, die terminale Säuregruppen aufweisen. Geeignete Dicarbonsäuren sind auch solche, die mit einer oder zwei Hydroxygruppen substituiert sind.

Erfindungsgemäß werden zur Teilneutralisierung bevorzugt Dicarbonsäuren, die einen ersten pKₛ-Wert von größer 2 und einen zweiten pKₛ-Wert von größer 4 aufweisen, eingesetzt. Besonders bevorzugt werden Dicarbonsäuren eingesetzt, die einen ersten pKₛ-Wert von größer 2,5 und einen zweiten pKₛ-Wert von größer 5 aufweisen. Der pKₛ-Wert ist der negative dekadische Logarithmus der Säurekonstante, wobei die Säurekonstante bei 25 °C und Atmosphärendruck gemeint ist.

Geeignete Dicarbonsäuren, die neben den Säuregruppen keine weiteren Substituenten tragen sind die gesättigten Alkandicarbonsäuren Malonsäure, Bernsteinsäure, Glutarsäure, Adipinsäure oder Sebacinsäure. Als mit ein oder zwei Hydroxygruppen substituierte Alkandicarbonsäuren eignen sich Äpfelsäure (2-Hydroxy-Bernsteinsäure) oder Weinsäure (2,3-Dihydroxybernsteinsäure). Als ungesättigte Dicarbonsäure eignet sich vor allem Fumarsäure.

Es können auch Mischungen solcher Dicarbonsäure verwendet werden.
So kann es sich empfehlen, Dicarbonsäuren, die eine besonders gute Resistenz der Überzüge erzeugen mit solchen Säuren, die eine besonders gute Redispergierbarkeit der Pulver erzeugen, zu mischen. Geeignete Mischungen sind z. B Adipinsäure mit Schwefelsäure bzw. Bernsteinsäure mit Oxalsäure.

Teilneutralisation im Sinne der Erfindung bedeutet, das 2 bis15, bevorzugt 4 bis 10 mol-% der Diethylaminoethylgruppen in Salzform vorliegen.

Bezüglich der Herstellung der als Filmbildner in den Überzügen dienenden Copolymere aus Methylmethacrylat und N,N-Diethylaminoethyl-methacrylat durch Emulsionspolymerisation wird ausdrücklich auf die Offenbarung der WO 2009/016258 Bezug genommen.

Die in den Dispersionen enthaltenen Copolymere weisen vorzugsweise einen K-Wert (bestimmt nach Fikentscher an einer 1%igen Lösung in N-Methylpyrrolidon (NMP)) im Bereich von 40 bis 60 auf.

Die Glasübergangstemperatur T_{G} bestimmt mittels DSC "Differential Scanning Calorimetry" liegt vorzugsweise in einem Bereich von 40 bis 70 °C, besonders bevorzugt 52 bis 62 °C. Dabei werden die Proben zunächst auf 150 °C aufgeheizt und dann von 150°C schnell abgekühlt. Die Messung der Glasübergangstemperatur erfolgt mit einer Heizrate von 20°K/min.
Die Mindestfilmbildetemperatur wird nach der in der DIN ISO 2115 beschriebenen Methode bestimmt und liegt im Bereich von 40 bis 70°C, vorzugsweise 50 bis 65°C. Die Messgenauigkeit der Methode liegt im Bereich von plus/minus 5 °C.

Bei den in den Dispersionen enthaltenen Copolymeren handelt es sich im Wesentlichen um statistische Copolymere.

Der mittlere Teilchendurchmesser der in der Polymerdispersion enthaltenen Polymerteilchen (bestimmt mittels analytischer Ultrazentrifuge) liegt vorzugsweise in einem Bereich von 70 bis 200 nm, besonders bevorzugt von 80 bis 150 nm, insbesondere von 90 bis 130 nm. Die Teilchengrößenverteilung ist vorzugsweise im Wesentlichen unimodal.
Der LD-Wert der erfindungsgemäß verwendeten wässrigen Dispersionen, bestimmt an einer 0,01%igen Dispersion in Wasser (2,5 cm Küvette, weißes Licht) beträgt vorzugsweise wenigstens 70 %, besonders bevorzugt wenigstens 80 %. Die Bestimmung der Lichtdurchlässigkeit wird z. B. in Dieter Distler, Wässrige Polymerdispersionen, Wiley-VCH (1999), S. 40, beschrieben.

Besonders bevorzugt ist ein Copolymer, welches ein Gewichtsverhältnis von Methylmethacrylat (MMA) zu Diethylaminoethylmethacrylat (DEAEMA) von 55:45 aufweist. Ein solches Copolymer ist auch kommerziell als Kollicoat® Smartseal, Firma BASF SE, erhältlich.

Erfindungsgemäß werden dem Copolymerpulver oder der entsprechenden wässrigen Dispersion die eingangs genannten C3-C10- Dicarbonsäuren zugesetzt. Vorzugsweise werden solche Mengen an Säure zugesetzt, dass die basischen Gruppen teilweise in Form der Säuresalze vorliegen. So liegen 1 bis 15 mol-% der basischen Gruppen in Salzform vor, besonders bevorzugt werden 5 bis 10 mol-% der basischen Gruppen neutralisiert.

Die Teilneutralisation des Copolymers aus MMA/DEAMA kann sowohl in der wässrigen Primärdispersion erfolgen, als auch am Pulver oder während oder nach wässriger Redispergierung eines Pulvers. Das Copolymer in Pulverform kann durch Sprühverfahren oder durch Gefriertrocknung erhalten werden.

So können beispielsweise die Dicarbonsäuren vor einer Sprühtrocknung zu der wässrigen Polymerdispersion gegeben werden. Erfolgt die Einarbeitung der Säure vor der Sprühtrocknung, so kann diese mit üblichen Verfahren in die wässrige Dispersion eingerührt werden. Gemäß einer weiteren Ausführungsform kann eine teilneutralisierte wässrige Primärdispersion durch Gefriertrocknung in die Pulverform überführt werden.

Gemäß einer anderen Ausführungsform kann die Säure auch vor oder während der Redispergierung eines zuvor aus der Primärdispersion hergestellten Pulvers zugesetzt werden. Bei Zugabe zu einem Pulver kann die Einarbeitung der Säure in das Polymerpulver so erfolgen, dass zunächst das Polymerpulver in Wasser mittels eines einfachen Rührers grob vordispergiert wird, anschließend die Säure zugesetzt wird und durch Weiterrühren die vollständige Redispergierung erreicht wird. Die Redispergierung ist üblicherweise sehr schnell und so liegen schon nach 10 min feinteilige Dispergate vor. In einer modifizierten Vorgehensweise kann auch zunächst die Säure in Wasser vorgelegt werden und hierzu unter Rühren das Polymerpulver gegeben werden. Gemäß einer weiteren Ausführungsform der Erfindung wird zunächst das Polymerpulver und die Säure gemischt und diese Pulvermischung in Wasser eingetragen. Gemäß dieser Ausführungsform kann man auch zunächst ein vollständig neutralisiertes Polymerpulver herstellen, welches anschließend durch Vermischen mit einem nicht neutralisierten Polymerpulver auf den gewünschten Teilneutralisationsgrad eingestellt wird.

Welche Gewichtsmengen an Säuren im Einzelnen verwendet werden, richtet sich nach dem jeweiligen Molekulargewicht der C3-C10-Dicarbonsäure und dem angestrebten Neutralisationsgrad.

Bevorzugt wird die Behandlung mit Säuren so durchgeführt, dass der pH-Wert der wässrigen Dispersion, des Pulvers oder des in Wasser redispergierten Pulvers im Bereich von 5 bis 9 liegt.

Besonders bevorzugt erfolgt die Zugabe der Säure oder des sauren Salzes so, dass der pH-Wert der wässrigen Dispersion, des Pulvers oder des in Wasser redispergierten Pulvers im Bereich von 6 bis 8 liegt.

Wie bereits erwähnt betreffen Ausführungsformen der Erfindung die Teilneutralisierung der Copolymere aus MMA/DEAMA in Pulverform. Bevorzugte Verfahrensweisen zur Herstellung der Pulverform der Copolymeren sind Sprühverfahren.
Der Feststoffgehalt der erfindungsgemäß für die Sprühverfahren eingesetzten Dispersionen beträgt vorzugsweise 10 bis 50 Gew.-%, besonders bevorzugt 20 bis 40 Gew.-%. Im Falle einer vorherigen Reinigung der Dispersion mittels Ultrafiltration weisen die erfindungsgemäß eingesetzten Dispersionen vorzugsweise vor und nach der Ultrafiltration Feststoffgehalte auf, die in diesen Bereichen liegen. Selbstverständlich ist es ebenfalls möglich, eine verdünnte Polymerdispersion vor dem Sprühverfahren einer Aufkonzentrierung durch Ultrafiltration zu unterziehen.

Vorzugsweise werden die zur Herstellung der erfindungsgemäßen Überzüge verwendeten Überzugsmittel durch Redispergierung in Wasser erhalten, wobei das durch ein Sprühverfahren erhaltene Pulver unter Einsatz von niedrig-scherenden Rührvorrichtungen bei Umdrehungen bis 1000 Upm redispergiert wird. Überraschenderweise können auch hoch-scherende Dispergiervorrichtungen bei Umdrehungen von > 5000 Upm verwendet werden. Dies kann erfindungsgemäß erfolgen, ohne dass die bei der Redisperdierung gebildeten feinen Teilchen agglomerieren und die Zubereitung koaguliert.

Die mittlere Teilchengröße des in Wasser redispergierten Polymerpulvers beträgt maximal das 5-fache, bevorzugt maximal das 3-fache, besonders bevorzugt maximal das 2-fache, der zugrundeliegenden Primärdispersion.

Mittlere Teilchengrößen beziehen sich dabei auf den Z-Average-Wert, der durch Lichtstreuung mittels eines "Malvern Zetasizer nano S" als Z-Average-Wert bestimmt wird.

Die Herstellung von Überzugsmitteln kann z. B. durch inniges Vermischen einer durch Redispergieren der erfindungsgemäß erhaltenen Polymerpulver zu einer wässrigen Polymerdispersion, der vorzugsweise mindestens ein weiterer Hilfsstoff zugesetzt wird, erfolgen.

Die Überführung der wässrigen Polymerdispersionen in die Pulverform kann mittels Sprühverfahren erfolgen. Als Sprühverfahren eignen sich prinzipiell die Sprühtrocknung, die agglomerierende Sprühtrocknung, die Sprühgranulation (Sprühwirbelschichttrocknung) oder die Sprühagglomeration.

Die im Folgenden genannten Bedingungen für die Durchführung der Zerstäubung und Trocknung beziehen sich auf alle prinzipiell durchführbaren Ausführungsformen des Sprühverfahrens, sei es normale Sprühtrocknung, Sprühgranulation oder agglomerierende Sprühtrocknung.

Die Zerstäubung erfolgt vorzugsweise als hydrodynamische Zerstäubung durch Flüssigkeitsdruck oder Luftdruck über Düsen wie beispielsweise Einstoff- oder Mehrstoff-Düsen oder über Zerstäubungsscheiben.

Als Sprühvorrichtungen eignen sich herkömmliche Sprühtürme, in die die zu zerstäubende Polymerdispersion von oben eingeführt wird. Die erhaltenen Polymerpulver können am unteren Ende ausgetragen werden und in einem nachgeschalteten Zyklon vom Trocknungsgasstrom abgeschieden werden.

Als Trocknungsgase können Luft oder inerte Gase wie Stickstoff, Argon oder Helium verwendet werden. Die Trocknungsgase können im Gegenstrom oder im Gleichstrom zu den durch die Zerstäubung erzeugten Flüssigkeitströpfchen durch die Sprühvorrichtung geleitet werden. Vorzugsweise wird das Trocknungsgas im Gleichstrom eingesetzt. Die Eingangstemperatur des Trocknungsgases wird mindestens 20 °C, vorzugsweise mindestens 40 °C über der Glasübergangstemperatur und gemäß einer Ausführungsform auch mindestens 20°C bevorzugt mindestens 40 °C, über der dynamischen Einfriertemperatur und mindestens 20°C, bevorzugt mindestens 40 °C, über der Mindestfilmbildetemperatur des Polymers gehalten. Die Eingangstemperatur des Trocknungsgases in die Sprühvorrichtung wird besonders bevorzugt bei 100 bis 140 °C und die Ausgangstemperatur des Trocknungsgases aus der Sprühvorrichtung bei 45 bis 70 °C gehalten. Ganz besonders bevorzugt wird die Eingangstemperatur des Trocknungsgases in die Sprühvorrichtung bei 110 bis 130 °C und die Ausgangstemperatur des Trocknungsgases aus der Sprühvorrichtung bei 50 bis 60 °C gehalten. Die Ausgangstemperatur des Trocknungsgases liegt ganz besonders bevorzugt im gleichen Temperaturbereich plus/minus 5 °C wie die Mindestfilmbildetemperatur.

Die Verdampfung des Wassers in der Sprühvorrichtung kann sowohl bei Normaldruck als auch bei 0.06 bis 0.12 MPa erfolgen.

Bei der Durchführung der Sprühverfahren können den wässrigen Polymerdispersionen auch polymere Sprühhilfsmittel wie Polyvinylalkohole, Mischungen aus Polyvinylalkohol und einem Pfropfpolymer aus Polyethylenglykol als Pfropfgrundlage und Polyvinylalkohol-Seitenketten (kommerziell verfügbar als Kollicoat® Protect), Polyvinylpyrrolidone,alkylierte und/oder hydroxyalkylierte Cellulosen, Stärkederivate, Ligninsulfonate, Polyacrylsäuren oder Polyacrylamide zugegeben werden. Geeignete Mengen solcher Sprühhilfsmittel liegen im Bereich von 0,1 bis 30, vorzugsweise 1 bis 10 Gew.-%, bezogen auf den Feststoffgehalt.

Weiterhin können den wässrigen Polymerdisperionen auch Antiblockiermittel zugegeben werden. Geeignete Antiblockiermittel sind z.B. Aluminium-Silikate wie Bentonit, weiterhin Kieselgur, kolloidale Kieselsäure, gefällte Kieselsäure, Diaatomeenerde, Calciumcarbonat, Titandioxid, Zinkoxid, Magnesiumsilikate wie Talkum oder Tricalciumphosphat. Geeignete Mengen solcher Antiblockiermittel liegen im Bereich von 0,1 bis 15, vorzugsweise 0,5 bis 5 Gew.-%, bezogen auf den Feststoffgehalt.

Prinzipiell können den wässrigen Polymerdispersionen auch übliche Coatinghilfsstoffe hinzugefügt werden. Geeignete Hilfsstoffe können sein: Aromastoffe, geschmacksverbessernde Stoffe, Süßungsmittel (Zucker, Zuckeralkohole, Süßstoffe wie z. B. Aspartame, Saccharin-Na, Natriumcyclamat), Gleitmittel, Netzmittel, Trennmittel, Antiklebemittel, Stabilisatoren, Antioxidantien, Porenbildner, Neutralisierungsmittel, Glanzmittel, Farbstoffe, Pigmente, Desinfektions- oder Konservierungsmittel, Verdickungsmittel oder Weichmacher. Geeignete Hilfsmittel sind z. B. in Fiedler, H. P. Lexikon der Hilfsstoffe für Pharmazie, Kosmetik und angrenzende Gebiete, 4. Aufl., Aulendorf: ECV-Editio-Cantor-Verlag, 1996, beschrieben.

Weiterhin kann die Überführung in Pulver durch eine Sprühgranulierung erfolgen. Dazu wird die zu trocknende wässrige Polymerdispersion ebenfalls zerstäubt und die erzeugten Tröpfchen gelangen dann in einem Wirbelbett in Kontakt mit vorgelegten Saatpartikeln, Durch dieses Inkontaktbringen der Saatpartikel mit den Tröpfchen der wässrigen Polymerdispersion wachsen die Saatteilchen zu größeren Granulatteilchen heran, wobei sich eine zwiebelschalenartige Struktur um das das als Saatgut verwendete Teilchen ausbildet.

Die Überführung in die Pulverform kann auch mit Hilfe der agglomerierenden Sprühtrocknung erfolgen. Dabei wird die Polymerdispersion in einem Sprühturm wie oben beschrieben zerstäubt, wobei gleichzeitig Feinstaub, der aus der Trocknungszone abgeführt wird, in die Zerstäubungszone, in der die wässrige Polymerdispersion in Form von feinen Tröpfchen vorliegt, eingeblasen wird. Die Feinstaubpartikel werden dabei zu größeren Aggregaten mit einer brombeerförmigen Struktur verklebt. Zusätzlich kann auch noch ein Wirbelbett angeschlossen werden, in dem der Wassergehalt der gebildeten Teilchen weiter reduziert werden kann. Die resultierenden Aggregate können Teilchengrößen von 150 bis 1000 µm bevorzugt von 200 bis 500 µm aufweisen. Auch bei dieser Ausführungssform wird die Eingangstemperatur mindestens 20 °C und bevorzugt mindestens 40 °C über der Glasübergangstemperatur, und gemäß einer Ausführungsform auch mindestens 20°C, bevorzugt mindestens 40 °C, über der dynamischen Einfriertemperatur und mindestens 20°C bevorzugt mindestens 40 °C über der Mindestfilmbildetemperatur des Polymers und die Ausgangstemperatur des Trocknungsgases aus der Sprühvorrichtung bei 40 bis 85 °C vorzugsweise bei 45 bis 70°C gewählt. Bevorzugt wird die Eingangstemperatur des Trocknungsgases in die Sprühvorrichtung bei 100 bis 140 °C und die Ausgangstemperatur des Trocknungsgases aus der Sprühvorrichtung bei 45 bis 70 °C gehalten. Besonders bevorzugt wird die Eingangstemperatur des Trocknungsgases in die Sprühvorrichtung bei 110 bis 130 °C und die Ausgangstemperatur des Trocknungsgases aus der Sprühvorrichtung bei 50 bis 60 °C gehalten. Die durch Sprühagglomeration erhaltenen brombeerförmigen Strukturen sind nahezu staubfrei und zeigen ein besonders vorteilhaftes Verhalten bei der Redispergierung.

Bei allen vorstehend genannten Ausführungsformen können während des Sprühprozesses Sprühhilfsmittel wie z.B. Aluminium-Silikate wie Bentonit, Kieselgur, kolloidale Kieselsäure, gefällte Kieselsäure, Diaatomeenerde, Calciumcarbonat, Titandioxid, Zinkoxid, Magnesiumsilikate wie Talkum oder Tricalciumphosphat in Mengen von 0,1 bis 15, vorzugsweise 0,5 bis 5 Gew.-%, bezogen auf das Polymerpulver in den Sprühturm eingeblasen werden.

Insgesamt richten sich die Teilchengrößen des durch Sprühverfahren entstehenden Pulvers nach der jeweiligen Ausführungsform. Bei einer normalen Sprühtrocknung können Teilchengrößen von 10 bis 150 µm erzielt werden. Bei einer Sprühgranulation wie beispielsweise einer Sprühwirbelschichttrocknung können größere Teilchengrößen von 150 bis zu 1000 µm erreicht werden. Bei der agglomerierenden Sprühtrocknung können Teilchengrößen von 150 bis 1000 µm erzielt werden.

Die Copolymeren werden als freifliessende Pulver erhalten, was im Sinne der vorliegenden Erfindung bedeutet, dass die Pulver bei der Bestimmung der Fließfähigkeit nach der DIN ISO 4324 unter Verwendung des Gerätes nach Pfrengle ohne Rührhilfe frei und vollständig aus dem Trichter ausfließen.
Der Restlösemittelgehalt beträgt üblicherweise nicht mehr als 5 Gew.-%, bezogen auf den Feststoffgehalt des Pulvers.

Die erfindungsgemäß zur Herstellung der Überzüge zu verwendenden redispergierbaren Copolymerpulver weisen in Wasser bei einem Feststoffgehalt von 20 Gew.-% niedrige Viskositäten von vorzugsweise kleiner 300 mPas, besonders bevorzugt kleiner 200 mPas und insbesondere kleiner 100 mPas auf (Werte bestimmt mittels Brookfield-Viskosimeter bei 20 °C und 100 s⁻¹). Solche Viskositäten sind für viele Anwendungen von besonderer Bedeutung.

Zur Stabilisierung können die Polymerdispersionen vor der Überführung in die Pulverform wie erwähnt mit in Wasser schwerlöslichen Antioxidantien behandelt werden. Der Begriff "Antioxidantien" ist dem Fachmann an sich bekannt (siehe z.B. Römpp - Lexikon der Chemie, 9. Auflage, 1989, Georg-Thieme-Verlag, Stuttgart) und bezeichnet Substanzen, die unerwünschte durch Sauerstoff oder andere oxidative Prozesse bedingte Veränderungen hemmen oder verhindern sollen. Als Antioxidantien zur Stabilisierung der Überzugsmittel eignen sich erfindungsgemäß in Wasser schwerlösliche Antioxidantien, also Antioxidantien deren Löslichkeit in Wasser bei 20 °C nicht mehr als 1 g/l beträgt.
Dabei eignen sich als Antioxidantien vor allem die lipophilen Substanzen Tocopherol, Tocopherolacetat, Ascorbylpalmitat, Ascorbylstearat, t-Butylhydrochinon, t-Butylhydroxyanisol, t-Butylhydroxytoluol, Octylgallat oder Dodecylgallat oder deren Kombinationen.

Dabei können die Antioxidantien auch in einem organischen Lösungsmittel gelöst eingesetzt werden. Als organische Lösungsmittel eignen sich solche Lösungsmittel, die einerseits ausreichend mit Wasser mischbar sind, so dass eine Konzentration von mindestens 10 Gew.-% in Wasser erreicht werden kann, , andererseits aber auch in der Lage sind, die in Wasser schwerlöslichen Antioxidantien lösen. Als Lösungsmittel in Betracht kommen Alkohole wie z.B. Ethanol oder Isopropanol, Ketone wie z.B. Aceton, Methylethylketon und Ester wie z.B. Methylacetat. Üblicherweise weisen diese Lösungsmittel Siedepunkte unter 100°C auf.
Die Antioxidantien können auf an sich übliche Weise in organische Lösung gebracht werden. Die Konzentration wird so gewählt, dass 10 bis 1000g Antioxidans pro Liter Lösungsmittel eingesetzt werden. Insgesamt wird die Menge an organischem Lösungsmittel so gewählt, dass 1 bis 20 Gew.-% Lösungsmittel bezogen auf das Gewicht der wässrigen Dispersion eingesetzt werden.

Gemäß einer weiteren Ausführungsform können die Antioxidantien in Form einer wässrigen micellaren Lösung in die wässrige Dispersion eingearbeitet werden. Dazu werden die Substanzen in Gegenwart von solubilisierend wirkenden Substanzen ("Solubilisatoren") in Lösung gebracht (zum Begriff der "Solubilisierung" siehe Römpp-Chemielexikon, 9. Auflage). Als Solubilisatoren eigenen sich Tenside wie z.B. Natrium-Docusat oder Natriumdodecylsulfat, ethoxylierte Fette, ethoxylierte Fettsäuren, ethoxylierte Fettalkohole oder polymere Solubilisatoren.

Als polymere Solubilisatoren eignen sich vor allem amphiphile Copolymere. Unter amphiphilen Copolymeren werden erfindungsgemäß Copolymere verstanden, die aus hydrophilen und hydrophoben Segmenten aufgebaut sind. Die Segmente können auch eine LCST (Lower Critical Solution Temperature) aufweisen. Die Segmente stellen ihrerseits Polymerketten dar, die aufgrund ihrer Zusammensetzung bzw. der zur Herstellung der Segmente verwendeten Monomeren entweder hydrophil oder hydrophob sind. Die amphiphilen Copolymere können Blockpolymere oder Pfropfpolymere sein. Die Struktur der Copolymer kann neben linearen Blockpolymeren auch kammförmig oder sternförmig sein. Im Falle der Pfropfpolymere können entweder hydrophobe Seitenketten und eine hydrophile Pfropfgrundlage vorliegen oder hydrophile Seitenketten und eine hydrophobe Pfropfgrundlage. Die Seitenketten können sowohl angepfropft als auch aufgepfropft werden. Geeignete amphiphile Copolymere sind beispielsweise in der WO 2007/017452, der WO 2007/051743, der WO 2007/ 065845 und der WO 2007/065846 offenbart, auf deren Beschreibung hinsichtlich geeigneter amphiphiler Copolymere und deren Herstellung hiermit Bezug genommen wird. Weitere amphiphile Copolymere sind beispielsweise Poloxamere.
Als hydrophile Segmente eigenen sich N-Vinyllactam-Homo- oder Copolymerketten, insbesondere N-Vinylpyrrolidon-haltige Polymere ebenso wie Polyvinylalkohol-Ketten oder Polyether. Als hydrophobe Segmente eignen sich beispielsweie Homo-oder Copolymere des N-Vinylacetats. Als Comonomer eignet sich beispielsweise N-Vinylcaprolactam. Bevorzugt wird als polymerer Solubilisator ein kommerziell unter dem Namen Soluplus®, Firma BASF SE, erhältliches Pfropfcopolymer mit PEG 6000 als Pfropfgrundlage und einer Copolymerseitenkette hergestellt aus Vinylacetat und N-Vinylcaprolactam. Weiterhin eignen sich zur Herstellung der micellaren Lösung alle Tenside, die einen HLB von über 12 aufweisen. Solche Tenside sind in "Fiedler, Encyclopedia of Excipients", Editio Cantor Verlag. Sixth edition, 2007, Seite 112 - 119, beschrieben. Die wässrigen Antioxidans - Solubilisate enthalten 0,5 bis 30 Gew.-%, vorzugsweise 1 bis 20 Gew.-% Antioxidans und 1 bis 50 Gew.-%, vorzugsweise 1 bis 30 Gew.-% Solubilisator. Insgesamt wird die Menge so gewählt, dass 1 bis 40 Gew.-% wässriges Antioxidans - Solubilisat bezogen auf das Gewicht der wässrigen Dispersion eingesetzt werden

Weiterhin können die in Wasser schwerlöslichen Antioxidantien in Form von feinteiligen wässrigen Dispersionen in die wässrige Dispersion des polymeren Überzugsmittels eingebracht. Als Dispersionen sind in diesem Zusammenhang zweiphasige Systeme bezeichnet, die entweder fest/flüssig (Suspensionen) oder flüssig/flüssig (Emulsionen) sein können. Die mittlere Teilchengröße (d4,3) der Antioxidantien sollte hierbei kleiner 20 µm, vorzugsweise kleiner 10 µm besonders bevorzugt kleiner 3 µm betragen.

So können die Antioxidantien in Emulgatoren gelöst und dann in Wasser dispergiert werden. Die Antioxidantien können aber auch direkt in Wasser gegeben werden und unter Zuhilfenahme von Emulgatoren mit hochscherenden Dispergierwerkzeugen dispergiert werden. Besonders bevorzugt ist hierbei die Erwärmung der Zubereitung auf eine Temperatur oberhalb des Schmelzpunktes des Antioxidans, wodurch eine Emulsion entsteht. Diese heiße Emulsion kann direkt unter Rühren zur Polymerdispersion hinzugefügt werden. Alternativ kann sie auch vorher abgekühlt werden, wodurch eine feinteilige Suspension entsteht. Besonders bevorzugt ist, die heiße Emulsion zu einer Polymerdispersion zu geben, die ebenfalls eine Temperatur oberhalb des Schmelzpunktes des Antioxidans aufweist.
Als Emulgatoren eignen sich prinzipiell alle Klassen von grenzflächenaktiven Substanzen mit einem HLB-Wert von > 10 (zum Hydrophilic-Lipophilic-Balance-Wert siehe Fiedler, Encyclopedia of Excipients, Editio Cantor Verlag Sixth edition, 2007, Seite 112 - 119). Als Emulgatoren eignen sich grundsätzlich alle ethoxilierten Fettsäuren, ethoxilierte Fettalkohole, ethoxilierte Fettsäureether oder ethoxilierte Fettsäureester mit entsprechenden HLB-Werten. Geeignet sind beispielsweise entsprechende ethoxilierte Sorbitan-, Stearyl, Oleyl-, Lauryl- oder Palmityl-Derivate, beispielsweise Solutol® HS (Macrogol 15 hydroxystearat) oder ethoxiliertes hydriertes Ricinusöl wie beispielsweise Cremophor® RH40 (ethoxiliert mit 40 Ethylenoxid-Einheiten) oder die entsprechenden Eumulgin®-Typen.
Weiterhin eignen sich als Emulgatoren Poloxamere (Polyethylenoxid-Polypropylenoxid-Blockcopolymere)

Die wässrigen Antioxidans - Emulgatordispersionen enthalten 1 bis 50 Gew.-%, vorzugsweise 2 bis 30 Gew.-% Antioxidans und 0,1 bis 30 Gew.-%, vorzugsweise 0,5 bis 10 Gew.-% Emulgator.
Insgesamt wird die Menge so gewählt, dass 1 bis 40 Gew.-% wässrige Antioxidans - Emulgatordispersion bezogen auf das Gewicht der wässrigen Polymerdispersion eingesetzt werden.

Weiterhin können die Antioxidantien in Form einer sogenannten "festen Lösung" eingesetzt. Der Begriff "feste Lösung" ist dem Fachmann bekannt und bezeichnet eine molekulardisperse Verteilung eines Feststoffs in einem anderen Feststoff. Im vorliegenden Falle können die Antioxidantien als feste Lösungen in einen geeigneten festen Solubilisator oder in ein polymeres Schutzkolloid eingearbeitet werden. Die resultierende feste Lösung kann dann direkt in fester Form in die wässrige Überzugsmitteldispersion eingearbeitet werden oder vorher in eine micellare wässrige Lösung oder in eine kolloidale Lösung überführt und dann in die wässrige Überzugsmitteldispersion eingearbeitet werden. Die festen Lösungen können beispielsweise hergestellt werden, indem die Antioxidantien gemeinsam mit dem Solubilisator oder dem Schutzkolloid in einem geeigneten Lösungsmittel gelöst und anschliessend das Lösungsmittel verdampft wird.

Weiterhin kann die feste Lösung der Antioxidantin durch Schmelzextrusion hergestellt, wobei Antioxidantien und Solubilisatoren oder polymere Schutzkolloide gemeinsam aufgeschmolzen und anschliessend extrudiert, geformt und verfestigt werden. Die nach der Extrusion erhaltenen granulären festen Schmelzextrudate lassen sich besonders vorteilhaft in die wässrige Dispersion des polymeren Überzugsmittels einarbeiten. Als Matrixpolymere und Schutzkolloide für feste Lösungen eignen sich hierbei die bereits erwähnten amphiphilen Copolymere, insbesondere Soluplus®, oder Poloxamere wie Lutrol® F86 aber auch nicht amphiphile Polymere wie z.B. Polyvinylpyrrolidone, Vinylpyrrolidon - Vinylacetat-Copolymere, Polyethylenglykole. Polyvinylalkohole, Polyvinylalkohol-Polyethylenglykol-Pfropfcopolymere oder hydroxyalkylierte Cellulosen.

Die Herstellung von Überzugsmitteln kann z. B. durch inniges Vermischen einer durch Redispergieren der erfindungsgemäß erhaltenen Polymerpulver zu einer wässrigen Polymerdispersion, der vorzugsweise mindestens ein weiterer Hilfsstoff zugesetzt wird, erfolgen.

Geeignete zusätzliche Hilfsstoffe können sein: Aromastoffe, geschmacksverbessernde Stoffe, Süßungsmittel (Zucker, Zuckeralkohole, Süßstoffe wie z. B. Aspartame, Saccharin-Na, Natriumcyclamat), Gleitmittel, Netzmittel, Trennmittel, Antiklebemittel, Stabilisatoren, Antioxidantien, Porenbildner, Neutralisierungsmittel, Glanzmittel, Farbstoffe, Pigmente, Desinfektions- oder Konservierungsmittel, Verdickungsmittel, Weichmacher etc. Solche Stoffe sind z. B. in Fiedler, H. P. Lexikon der Hilfsstoffe für Pharmazie, Kosmetik und angrenzende Gebiete, 4. Aufl., Aulendorf: ECV-Editio-Cantor-Verlag, 1996, beschrieben.

Zur Herstellung des Überzugsmittels kann das MMA/DEAEMA-Polymerpulver vor der Redispergierung in Wasser gemahlen werden. Die Mahlung kann auch in Gegenwart der genannten zusätzlichen Hilfsstoffe erfolgen.

Übliche Mengen der Hilfsstoffe liegen in einem Bereich von jeweils 0 bis 70 Gew.-%, bevorzugt 0 bis 60 Gew.-%, insbesondere 1 bis 50 Gew.-%, bezogen auf das Gesamtgewicht des Feststoffes des Überzugsmittels.

Gemäß einer Ausführungsform der Erfindung erfolgt die Herstellung der Überzüge mit einem erfindungsgemäßen Überzugsmittel in wässriger Form. Der Auftrag des Überzugsmittels kann in wässriger Form durch Granulieren, Gießen, Ausstreichen oder mittels Sprühauftrag erfolgen.

Das erfindungsgemäß erhaltene Überzugsmittel kann auch in Pulverform auf die pharmazeutischen Dosierungsformen aufgetragen werden.

Bevorzugt ist die Anwendung als durch Redispergierung eines pulverförmigen erfindungsgemäßen Überzugsmittels erhaltene wässrige Polymerdispersion. Grundsätzlich eignet sich jede Dispergiervorrichtung für die Redispergierung. Dabei erfolgt die Redispergierung vorzugsweise unter Anwendung niedriger Scherkräfte, vorzugsweise mittels eines Blatt, Propeller, Ankerrührers oder eines vergleichbaren Rührwerkzeuges. Die erfindungsgemäßen Polymerpulver redispergieren hierbei spontan und schnell. Die Redispergierung der Polymerpulver in Wasser ist üblicherweise in 10 min abgeschlossen.

Diesen redispergierten Zubereitungen können für die Herstellung der Überzüge erforderliche weitere Komponenten zugesetzt werden. Solche Komponenten sind insbesondere Weichmacher wie z.B. Triethylcitrat, Tributylcitrat, Diethylsebacat, Dibutylsebacat, Acetyltriethylcitrat.

Die Herstellung der feinteiligen Dispersionen durch Redispergierung von Pulvern kann auch bei sehr hohen Scherkräften wie beispielsweise in einer Rotor-Stator-Vorrichtung, die auch Ultra-Turrax genannt wird, oder einer Kolloidmühle erfolgen. Die Eintragung hoher Scherkräfte wird in einer Rotor-Stator-Vorrichtung über die Umdrehungszahl der Vorrichtung geregelt. Vorzugsweise erfolgt die Redispergierung mit Hilfe einer Dispergiervorrichtung bei < 5000 Upm. Dieses Verfahren ist insbesondere von Vorteil wenn zusätzlich in die Dispersion weitere grobteilige Zusatzstoffe oder agglomerierte Zusatzstoffe eingearbeitet werden müssen, die einer besonderen Zerkleinerung bedürfen. Es entfällt somit die separate Zerkleinerung dieser Zusatzstoffe in Wasser und spätere Zugabe zum redispergierten Polymerpulver.

In einer besonderen Ausführungsform werden aus den erfindungsgemäßen redispergierbaren Polymerpulvern durch Vermischen mit weiteren üblichen vorstehend beschriebenen Coatingbestandteilen bzw. Zusatzstoffen sogenannte ready-to use Zubereitungen hergestellt, die alle erforderlichen Bestandteile eine fertigen Überzugs enthalten. Diese liegen in Pulver- oder Granulatform vor. Der Anwender braucht diese zur Herstellung einer sprühfertigen Suspension nur noch in Wasser einzurühren. Die Herstellung dieser ready-to-use Zubereitungen erfolgt durch trockenes Mischen, Vermahlen, Kompaktieren oder Granulieren der Bestandteile mit einer Granulierflüssigkeit gefolgt von einem Abtrocknungsschritt. So kann beispielsweise ein erfindungsgemäß teilneutralisiertes Polymerpulver mit einer wässrige Suspension, enthaltend Pigmenten und optional weitere Hilfsstoffen, granuliert werden. Diese Granulate können dann zu einer Sprühsuspension redispergiert werden.

Die erfindungsgemäßen Überzugsmittel können zusätzlich wenigstens eine weitere Polymerkomponente enthalten. Dabei können Mischungen aus wenigstens zwei Dispersionen, wenigstens einer Dispersion und wenigstens einer Lösung, wenigstens einer Dispersion und wenigstens einem Pulver, wenigstens zwei Pulvern, etc. zum Einsatz kommen.

Unabhängig von den einzelnen Ausführungsformen der Erfindung liegt die Auftragsmenge des Überzugsmittels bevorzugt im Bereich von 1 bis 20 mg/cm², bevorzugt bei 2 bis 15 mg/cm², besonders bevorzugt 4 bis 12 mg/cm².

Erfindungsgemäß dienen die Überzugsmittel zur Herstellung von Überzügen für pharmazeutische Dosierungsformen, die im sauren Milieu des Magens schnell freisetzend sein sollen. D.h. die erfindungsgemäßen Überzüge sind magensaftlöslich. Schnell freisetzend bedeutet in diesem Zusammenhang, dass nach 60 min bei 25 °C unter Atmosphärendruck nach dem Paddle-Modell (Medium: 0,1 N HCl) mindestens 80 % des Wirkstoffs freigesetzt sind. Erfindungsgemäß erhaltene Überzüge sollen sich in Mundhöhle und Rachen im neutralen oder nahezu neutralen Milieu des Speichels nicht auflösen.

Die erfindungsgemäßen Überzüge können zur Geschmacksmaskierung oder zum Schutz vor Feuchtigkeit verwendet werden. Die Wasserdampfpermeabilität der Überzüge ist sehr niedrig, wodurch feuchtigkeitsempfindliche Wirkstoffe geschützt werden.

Pharmazeutische Dosierungsformen, die mit den erfindungsgemäßen Überzügen versehen werden können, sind Tabletten, Kapseln oder Pellets. Weiterhin können auch Wirkstoffkristalle mit den erfindungsgemäßen Überzügen versehen werden.

Die nach dem erfindungsgemäßen Verfahren erhaltenen Überzugsmittel eignen sich für Dosierungsformen grundsätzlich beliebiger pharmazeutischer Wirkstoffe, die vorzugsweise in isolierter oder geschützter Form verabreicht werden können, wie Antidepressiva, Betarezeptorenblocker, Antidiabetika, Analgetika, Antiphlogistika, Antirheumatika, Antihypotonika, Antihypertonika, Psychopharmaka, Tranquilizer, Antiemetika, Muskelrelaxantien, Glucocorticoide, Mittel zur Behandlung von Colitis ulcerosa oder Morbus Crohn, Antiallergika, Antibiotika, Antiepileptika, Antikoagulantia, Antimykotika, Antitussiva, Arteriosklerosemittel, Diuretika, Enzyme, Enzyminhibitoren, Gichtmittel, Hormone und deren Hemmstoffe, Herzglykoside, Immuntherapeutika und Zytokine, Laxantien, Lipidsenker, Magen- Darmtherapeutika, Migränemittel, Mineralstoffpräparate, Otologika, Parkinsonmittel, Schilddrüsentherapeutika, Spasmolytika, Thrombozytenaggregationshemmer, Vitamine, Zytostatika und Metastasenhemmer, Phytopharmaka, Chemotherapeutika, Nutraceuticals, Vitamine, Carotinoide und Aminosäuren.

Beispiele geeigneter Wirkstoffe sind: Acarbose, Nichtsteroidale Antirheumatika, Herzglykoside, Acetylsalicylsäure, Virustatika, Aclarubicin, Aciclovir, Cisplatin, Actinomycin, α-und β-Sympatomimetika, Allopurinol, Alosetron, Alprostadil, Prostaglandine, Amantädin, Ambroxol, Amlodipin, Methotrexat, 5-Aminosalicylsäure, Amitriptylin, Amlodipin, Amoxicillin, Anastrozol, Atenolol, Atorvastatin, Azathioprin, Balsalazid, Beclomethason, Betahistin, Bezafibrat, Bicalutamid, Diazepam und Diazepamderivate, Budesonid, Bufexamac, Buprenorphin, Methadon, Calciumsalze, Kaliumsalze, Magnesiumsalze, Candesartan, Carbamazepin, Captopril, Cefalosporine, Celetoxib, Cetirizin, Chenodeoxycholsäure, Ursodeoxycholsäure, Theophyllin und Theophyllinderivate, Trypsine, Cimetidin, Clarithromycin, Clavulansäure, Clindamycin, Clobutinol, Clonidin, Cotrimoxazol, Codein, Coffein, Vitamin D und. Derivate von Vitamin D, Colestyramin, Cromoglicinsäure, Cumarin und Cumarinderivate, Cystein, Cytarabin, Cyclophosphamid, Ciclosporin, Cyproteron, Cytarabin, Dapiprazol, Desogestrel, Desonid, Dihydralazin, Diltiazem, Mutterkornalkaloide, Dimenhydrinat, Dimethylsulfoxid, Dimeticon, Dipyridamol, Domperidon und Domperidonderivate, Donepzil, Dopamin, Doxazosin, Doxorubicin, Doxylamin, Dapiprazol, Benzodiazepine, Diclofenac, Glykosidantibiotika, Desipramin, Econazol, ACE-Hemmer, Enalapril, Ephedrin, Epinephrin, Epoetin und Epoetinderivate, Morphinane, Calciumantagonisten, Irinotecan, Modafinil, Orlistat, Peptidantibiotika, Phenytoin, Riluzole, Risedronat, Sildenafil, Topiramat, Makrolidantibiotika, Esomeprazol, Estrogen und Estrogenderivate, Gestagen und Gestagenderivate, Testosteron und Testosteronderivate, Androgen und Androgenderivate, Ethenzamid, Etofenamat, Etofibrat, Fenofibrat, Etofyllin, Etoposid, Famciclovir, Famotidin, Felodipin, Fenofibrat, Fentanyl, Fenticonazol, Gyrasehemmer, Fluconazol, Fludarabin, Flunarizin, Fluorouracil, Fluoxetin, Flurbiprofen, Ibuprofen, Flutamid, Fluvastatin, Follitropin, Formoterol, Fosfomicin, Furosemid, Fusidinsäure, Galantamin, Gallopamil, Ganciclovir, Gemfibrozil, Gentamicin, Ginkgo, Johanniskraut, Glibenclamid, Harnstoffderivate als orale Antidiabetika, Glucagon, Glucosamin und Glucosaminderivate, Glutathion, Glycerol und Glycerofderivate, Hypothalamushormone, Goserelin, Guanethidin, Halofantrin, Haloperidol, Heparin und Heparinderivate, Hyaluronsäure, Hydralazin, Hydrochlorothiazid und Hydrochlorothiazidderivate, Salicylate, Hydroxyzin, Idarubicin, Ifosfamid, Imipramin, Indometacin, Indoramin, Insulin, Interferone, Jod und Jodderivate, Isoconazol, Isoprenalin, Glucitol und Glucitolclerivate, Itraconazol, Ketoconazol, Ketoprofen, Ketotifen, Lacidipin, Lansoprazol, Levodopa, Levomethadon, Schilddrüsenhormone, Liponsäure und Liponsäurederivate, Lisinopril, Lisurid, Lofepramin, Lomustin, Loperamid, Loratadin, Maprotilin, Mebendazol, Mebeverin, Meclozin, Mefenaminsäure, Mefloquin, Meloxicam, Mepindolol, Meprobamat, Meropenem, Mesalazin, Mesuximid, Metamizol, Metformin, Methotrexat, Methylphenidat, Methylprednisolon, Metixen, Metoclopramid, Metoprolol, Metronidazol, Mianserin, Miconazol, Minocyclin, Minoxidil, Misoprostol, Mitomycin, Mizolastin, Moexipril, Morphin und Morphinderivate ; Nachtkerze, Nalbuphin, Naloxon, Tilidin, Naproxen, Narcotin, Natamycin, Neostigmin, Nicergolin, Nicethamid, Nifedipin, Nifluminsäure, Nimodipin, Nimorazol, Nimustin, Nisoldipin, Adrenalin und Adrenalinderivate, Norfloxacin, Novaminsulfon, Noscapin, Nystatin, Ofloxacin, Olanzapin, Olsalazin, Omeprazol, Omoconazol, Ondansetron, Orlistat, Oseltamivir, Oxaceprol, Oxacillin, Oxiconazol, Oxymetazolin, Pantoprazol, Paracetamol, Paroxetin, Penciclovir, orale Penicilline, Pentazocin, Pentifyllin, Pentoxifyllin, Perphenazin, Pethidin, Pflanzenextrakte, Phenazon, Pheniramin, Barbitursäurederivate, Phenylbutazon, Phenytoin, Pimozid, Pindolol, Piperazin, Piracetam, Pirenzepin, Piribedil, Piroxicam, Pramipexol, Pravastatin, Prazosin, Procain, Promazin, Propiverin, Propranolol, Propyphenazon, Prostaglandine, Protionamid, Proxyphyllin, Quetiapin, Quinapril, Quinaprilat, Ramipril, Ranitidin, Reproterol, Reserpin, Ribavirin, Rifampicin, Risperidon, Ritonavir, Ropinirol, Rosiglitazon, Roxatidin, Roxithromycin, Ruscogenin, Rutosid und Rutosidderivate, Sabadilla, Salbutamol, Salmeterol, Scopolamin, Selegilin, Sertaconazol, Sertindol, Sertralin, Silikate, Simvastatin, Sitosterin, Sotalol, Spagluminsäure, Sparfloxacin, Spectinomycin, Spiramycin, Spirapril, Spironolacton, Stavudin, Streptomycin, Sucralfat, Sufentanil, Sulbactam, Sulfonamide, Sulfasalazin, Sulpirid, Sultamicillin, Sultiam, Sumatriptan, Suxamethoniumchlorid, Tacrin, Tacrolimus, Taliolol, Tamoxifen, Taurolidin, Tazaroten, Tegaserod, Temazepam, Teniposid, Tenoxicam, Terazosin, Terbinafin, Terbutalin, Terfenadin, Terlipressin, Tertatolol, Tetracycline, Tetryzolin, Theobromin, Theophyllin, Butizin, Thiamazol, Phenothiazine, Thiotepa, Tiagabin, Tiaprid, Propionsaurederivate, Ticlopidin, Timolol, Tinidazol, Tioconazol, Tioguanin, Tioxolon, Tiropramid, Tizanidin, Tolazolin, Tolbutamid, Tolcapon, Tolnaftat, Tolperison, Topotecan, Torasemid, Antiöstrogene, Tramadol, Tramazolin, Trandolapril, Tranylcypromin, Trapidil, Trazodon, Triamcinolon und Triamcinolonderivate, Triamteren, Trifluperidol, Trifluridin, Trimethoprim, Trimipramin, Tripelennamin, Triprolidin, Trifosfamid, Tromantadin, Trometamol, Tropalpin, Troxerutin, Tulobuterol, Tyramin, Tyrothricin, Urapidil, Ursodeoxycholsäure, Chenodeoxycholsäure, Valaciclovir, Valdecoxib, Valproinsäure, Vancomycin, Vecuroniumchlorid, Venlafaxin, Verapamil, Vidarabin, Vigabatrin, Viloxazin, Vinblastin, Vincamin, Vincristin, Vindesin, Vinorelbin, Vinpocetin, Viquidil, Warfarin, Xantinolnicotinat, Xipamid, Zafirlukast, Zalcitabin, Zanamivir, Zidovudin, Zolmitriptan, Zolpidem, Zopiclon, Zotepin und dergleichen.

Die Wirkstoffe können gewünschten falls auch in Form ihrer pharmazeutisch akzeptablen Salze oder Derivate verwendet werden, und im Falle chiraler Wirkstoffe können sowohl optisch aktive Isomere als auch Racemate oder Diastereoisomerengemische eingesetzt werden. Gewünschten falls können die erfindungsgemäßen Zusammensetzungen auch zwei oder mehrere pharmazeutische Wirkstoffe enthalten.

Erfindungsgemäß können die Überzugsmittel zur Beschichtung von Extrudaten, Minitabletten, Kapseln, Weichkapseln, Granulaten, Pellets, Mikropellets, Mikrokapseln, Nanokapseln oder Kristallen eingesetzt werden.

Zur Herstellung von Dosierungsformen können die überzogenen Granulate, Pellets, Mikropellets, Mikrokapseln, Kristalle mit geeigneten Hilfsstoffen abgemischt und zu Tabletten verpresst werden, die im wässrigen Milieu der Mundhöhle zerfallen und die gecoateten feinen Formlinge wieder freigeben. Besondere Bedeutung haben hierbei so genannte oral dispersibles, d. h. Tabletten, die im Mund innerhalb von kurzer Zeit zerfallen und die geschmacksmaskierten kleinen Formlinge freisetzen.

Weiterhin können die Überzugsmittel auch vorteilhaft zum Überziehen von Tabletten eingesetzt werden.

Wirkstoffklassen und Substanzen, die oftmals einen unangenehmen bitteren Geschmack hervorrufen können und sich vorteilhaft erfindungsgemäß formulieren lassen, sind z. B.:
Analgetika und Antirheumatika, wie Paracetamol, Diclofenac, Aceclofenac, Ibuprofen, Ketoprofen, Flubiprofen, Acetylsalicylsäure, Levacetylmethadol und Oxycodon;
Psychopharmaka, wie Promethazine, Donepezil, Modafinil, Nefazodon, Reboxetin, Sertindol und Sertralin;
Antibiotika, wie Erythromycin, Roxithromycin, Clarithromycin, Grepafloxacin, Ciprofloxacin, Levofloxacin, Sparfloxacin, Trovafloxacin und Nevirapin;
Betablocker, wie Propranolol, Metoprolol, Bisoprolol und Nebivolol;
Antidiabetika, wie Metformin, Miglitol und Repaglinid;
H₁-Antihistaminika, wie Diphenhydramin, Fexofenadin und Mizolastin;
H₂ Antihistaminika, wie Cimetidin, Famotidin, Roxatidin, Nizatidin, Ticlopidin, Cetirizin und Ranitidin;
Vitamine wie Thiaminnitrat sowie Chinidin-Sulfat, Amyloprilose-HCl, Pseudoephedrin-HCl, Sildenafil, Topiramat, Granisetron, Rebamipide, Chinin- HCl, etc.
Auch verschiedene Salze dieser Wirkstoffe können entsprechend formuliert werden.

Die hervorragende Geschmacksmaskierung resultiert aus der Unlöslichkeit der erfindungsgemäßen Polymeren bei pH-Werten größer 6 und der schnellen Löslichkeit bei pH-Werten unter 6. Das heißt im Speichel (pH-Wert: 7,2) sind entsprechend überzogene Formen sehr lange stabil und es erfolgt kein Kontakt des bitteren Arzneistoffs mit der Mundschleimhaut, aber im Magen bei pH-Werten von 1 bis 5 erfolgt eine sehr schnelle Freisetzung des Wirkstoffs. Die Auflösung ist dabei so schnell, dass kein Unterschied im Wirkungseintritt vorhanden ist, verglichen mit einer nicht gecoateten Form. In der Regel lösen sich Filmüberzüge eines erfindungsgemäßen Polymers innerhalb von 5 min in Magensaft auf, wohingegen sie in Phosphatpuffer pH 7,2 über 2 Stunden stabil sind. Überraschenderweise lösen sich die Filmüberzüge auch in Medien mit pH-Werten von 4,5 relativ schnell, so dass die daraus hergestellten Darreichungsformen auch bei anaciden Patienten bzw. Patienten, die mit Antacida behandelt werden, eine schnelle Wirkung entfalten. Diese hervorragenden Anwendungseigenschaften der Überzugsmittel bleiben auch nach der Überführung in Pulver und Redispergierung beziehungsweise Aufschmelzen der Pulver erhalten.

### Beispiele

Verwendete Abkürzungen:
Glasübergangstemperatur: Tg
VE-Wasser: vollentsalztes Wasser
Als Polymer wurde in allen Beispielen ein als Polymer A bezeichnetes Polymer eingesetzt. Die Herstellung des Polymeren A erfolgte analog Beispiel 1 der WO 2009/016258.
Polymer A : Methylmethacrylat/Diethylaminoethylmethacrylat, Gew.v. 55:45, K-Wert 49, Tg 57 °C

Der K-Wert wurde 0.1 gew.-%ig in NMP gemessen. Das Polymer wurde als wässrige Dispersion mit einem pH-Wert von 9+/- 0.3 eingesetzt oder als sprühgetrocknetes Pulver eingesetzt. Die mittlere Partikelgröße der Primärdispersion betrug 127 nm. Die Glasübergangstemperaturen wurden mittels DSC bei einer Heizrate von 20 °K/min bestimmt. Die Mindestfilmbildetemperatur entsprach im Rahmen der Messgenauigkeit von plus/minus 5 °C der Tg.

Bei der Bestimmung der mittleren Teilchengrößen der Pulver wurde der D(4,3)-Wert mittels Lichtbeugung mittels eines Malvern Mastersizers 2000 bestimmt.

Bei der Bestimmung der mittleren Teilchengrößen der redispergierten Pulver mittels Lichtstreuung wurde der Wert mittels eines "Malvern Zetasizer nano S" als Z-Average-Wert bestimmt.

Verwendete Hilfsstoffe:
Ludipress®: freifliessendes Granulat aus 94, 4 Gew.-% Lactose, 3,2 Gew.-% Kollidon 30 (USP) und 3,4 Gew.-% Kollidon CL (USP)
Kollidon® CL-F: Crospovidone
Avicel® PH102: mikrokristalline Cellulose, mittlerer Teilchendurchmesser 100 µm
Simethicon: CTFA-Bezeichnung für ein Gemisch aus Dimethicon mit einer durchschnittlichen Kettenlänge von 200-350 Dimethylsiloxan-Einheiten und Silicagel
Aerosil® 200: feinteilige Kieselsäure

### Beispiel 1 (Vergleichsbeispiel)

1000 ml einer wässrigen Dispersion des Polymers A mit einem Feststoffgehalt von 30 Gew.-% wurden unter Rühren mit 6,5 g 85 Gew.-%iger Phosphorsäure vermischt. Dies entspricht einem Neutralisationsgrad von 8 mol-%. Diese teilneutralisierte Sprühsuspension wurde in einem Sprühturm sprühgetrocknet. Die Zerstäubung erfolgte hierbei über eine 1,4 mm Zweistoffdüse mit 3,0 bar Zerstäubungsdruck. Das Trocknungsgas wurde tangential im Eingangsbereich des Sprühtrockners zugeführt und das getrocknete Produkt in einem Zyklon abgeschieden. Die Zulufttemperatur betrug 107°C und die Ablufttemperatur 56°C. Die mittlere Teilchengröße des Pulvers betrug 30 µm.
100 g des sprühgetrockneten Produkts wurde in Wasser zu einer Sprühsuspension mit 20% Feststoffgehalt durch Rühren mit einem Blattrührer für 60 min dispergiert. Die Messung der Teilchengröße mittels Lichtstreuung zeigte eine bimodale Verteilung mit einem Maximum sowohl bei 130 nm als auch bei 450 nm.
Die so hergestellte Zubereitung wurde mit 14 g Acetyltriethylcitrat versetzt, zwei Stunden gerührt und durch Versprühen auf Tablettenkerne aufgebracht.

**Tabelle 1: Zusammensetzung Kern:**

| Zuammensetzung | [%] | [mg] |
|---|---|---|
| Coffein, gran. 0,2-0,5mm | 15,15 | 50 |
| Ludipress | 72,43 | 239 |
| Avicel PH 101 | 12,12 | 40 |
| Magnesiumstearat | 0,3 | 1 |
| | 100,00 | 330 |

Zur Herstellung der Kerne wurden die abgewogenen Bestandteile Coffein, Ludipress und Avicel PH 101 in einem Diosna-Mischer für 3 Minuten auf Stufe 1 gemischt. Nach Zugabe von Magnesiumstearat wurde nochmals 1 Minute gemischt. Die so hergestellte Pulvermischung wurde auf einer Rundläuferpresse zu Tabletten mit einem Gewicht von 330 mg und einer Bruchfestigkeit von 80 N verpresst.

**Tabelle 2: Sprühbedingungen:**

| Equipment | Innojet Ventilus 1 Düse: **I**RN 2 |
|---|---|
| Zulufttemperatur | 60°C |
| Zuluftmenge | 90 m³/h |
| Ansatzgröße | 150 g |
| Sprührate | 1,5 g/min |
| Sprühdruck | 0.15 MPa |
| Auftragsmenge | 4mg/cm² |

Die Prüfung auf Resistenz der gecoateten Tabletten in Puffer pH 6,8 erfolgte mittels Sechsfachbestimmung in einem Freisetzungsgerät (USP, Apparatur 2).

Die Resistenz der Tabletten (Wirkstofffreisetzung < 2%) wurde nach 30, 60, 90 und 120 min bestimmt.
Die so hergestellten Tabletten zeigten keine Resistenz in Puffer pH 6,8.

### Beispiel 2 (Vergleichsbeispiel)

5,1 g Oxalsäure wurden in 500 ml VE-Wasser gelöst und anschließend unter Rühren in 1000 ml einer wässrigen Dispersion des Polymers A mit einem Feststoffgehalt von 30 Gew.-% eingearbeitet. Dies entspricht einem Neutralisationsgrad von 8 mol-%. Diese teilneutralisierte Sprühsuspension wurde analog Beispiel 1 in einem Sprühturm sprühgetrocknet.
Die mittlere Teilchengröße des Pulvers betrug 35 µm.
Das Pulver wurde analog Beispiel 1 redispergiert und zeigt eine monomodale Verteilung mit einem Maximum bei 180 nm.
Die Weiterverarbeitung zu Filmtabletten mit einer Auftragsmenge von 4 mg/cm² erfolgte analog Beispiel 1. Die so hergestellten Tabletten zeigten trotz sehr guter Redispergierung keine Resistenz in Puffer pH 6,8.

### Beispiel 3

6,7 g Bernsteinsäure wurden in 500 ml VE-Wasser gelöst und anschließend unter Rühren in 1000 ml einer wässrigen Dispersion des Polymers A mit einem Feststoffgehalt von 30 Gew.-% eingearbeitet. Dies entspricht einem Neutralisationsgrad von 8 mol-%. Diese teilneutralisierte Sprühsuspension wurde analog Beispiel 1 in einem Sprühturm sprühgetrocknet.
Die mittlere Teilchengröße des Pulvers betrug 34 µm.
Das Pulver wurde analog Beispiel 1 redispergiert und zeigt eine monomodale Verteilung mit einem Maximum bei 170 nm.
Die Weiterverarbeitung zu Filmtabletten mit einer Auftragsmenge von 4, 8 und 12 mg/cm² erfolgte analog Beispiel 1.
Die Prüfung der Tabletten auf Resistenz in Puffer pH 6,8 zeigte folgende Ergebnisse:

| | | | |
|---|---|---|---|
| | 4 mg/cm² | 8 mg/cm² | 12 mg/cm² |
| 30 min | 100 % | 100 % | 100 % |
| 60 min | 72 % | 95 % | 100 % |

### Beispiel 4 (Vergleichsbeispiel)

12,1 g Natriumdihydrogencitrat wurden in 500 ml VE-Wasser gelöst und anschließend unter Rühren in 1000 ml einer wässrigen Dispersion des Polymers A mit einem Feststoffgehalt von 30 Gew.-% eingearbeitet. Dies entspricht einem Neutralisationsgrad von 8 mol%. Diese teilneutralisierte Sprühsuspension wurde analog Beispiel 1 in einem Sprühturm sprühgetrocknet.
Die mittlere Teilchengröße des Pulvers betrug ca. 32 µm.
Das Pulver wurde analog Beispiel 1 redispergiert und zeigte eine bimodale Verteilung der Teilchengrößen mit einem Maximum sowohl bei 197 nm als auch bei 520 nm.

**Tabelle 3: Zusammensetzung Kern:**

| Zuammensetzung | [%] | [mg] |
|---|---|---|
| Propranolol-HCl | 13,4 | 40,0 |
| Ludipress® | 40,2 | 120,7 |
| Avicel® PH 102 | 40,2 | 120,7 |
| Kollidon® VA 64 | 5,2 | 15,6 |
| Magnesiumstearat | 1,0 | 3,0 |
| | **100,00** | **300** |

Zur Herstellung der Kerne wurden die abgewogenen Bestandteile Propranolol-HCl, Ludipress, Avicel PH 102 und Kollidon VA 64 in einem Diosna-Mischer für 3 Minuten auf Stufe 1 gemischt. Nach Zugabe von Magnesiumstearat wurde nochmals 1 Minute gemischt. Die so hergestellte Pulvermischung wurde auf einer Rundläuferpresse zu Tabletten mit einem Gewicht von 300 mg und einer Bruchfestigkeit von 85 N verpresst.
Die Weiterverarbeitung zu Filmtabletten mit einer Auftragsmenge von 4 mg/cm² erfolgte analog Beispiel 1.

Die so hergestellten Filmtabletten zeigten keine Resistenz in Puffer pH 6,8.

### Beispiel 5 (Vergleichsbeispiel)

168,5 ml 1 molare Schwefelsäure wurden in 500 ml VE-Wasser gelöst und anschließend unter Rühren in 1000 ml einer wässrigen Dispersion des Polymers A mit einem Feststoffgehalt von 30 Gew.-% eingearbeitet. Dies entspricht einem Neutralisationsgrad von 8 mol-%. Diese teilneutralisierte Sprühsuspension wurde analog Beispiel 1 in einem Sprühturm sprühgetrocknet.
Die mittlere Teilchengröße des Pulvers betrug ca. 37 µm.
Das Pulver wurde analog Beispiel 1 redispergiert und zeigt eine monomodale Verteilung mit einem Maximum bei 192 nm.
Die Weiterverarbeitung zu Filmtabletten mit einer Auftragsmenge von 4 mg/cm² erfolgte analog Beispiel 1. Die so hergestellten Tabletten zeigten überraschenderweise trotz sehr guter Redispergierung keine Resistenz in Puffer pH 6,8.

### Beispiel 6

7,4 g Glutarsäure wurden in 500 ml VE-Wasser gelöst und anschließend unter Rühren in 1000 ml einer wässrigen Dispersion des Polymers A mit einem Feststoffgehalt von 30 % eingearbeitet. Dies entspricht einem Neutralisationsgrad von 8 mol%. Diese teilneutralisierte Sprühsuspension wurde analog Beispiel 1 in einem Sprühturm sprühgetrocknet.
Die mittlere Teilchengröße des Pulvers betrug ca. 32 µm.

Das Pulver wurde analog Beispiel 1 redispergiert und zeigt eine monomodale Verteilung mit einem Maximum bei 178 nm.
Die Weiterverarbeitung zu Filmtabletten mit einer Auftragsmenge von 4, 8 und 12 mg/cm² erfolgte analog Beispiel 1.
Die Prüfung der Tabletten auf Resistenz in Puffer pH 6,8 zeigte folgende Ergebnisse:

| | | | |
|---|---|---|---|
| | 4 mg/cm² | 8 mg/cm² | 12 mg/cm² |
| 30 min | 100 % | 100 % | 100 % |
| 60 min | 68 % | 91 % | 100 % |

### Beispiel 7 (Vergleichsbeispiel)

15,9 g Stearinsäure wurden in 500 ml VE-Wasser gelöst und anschließend unter Rühren in 1000 ml einer wässrigen Dispersion des Polymers A mit einem Feststoffgehalt von 30 % eingearbeitet. Dies entspricht einem Neutralisationsgrad von 8 mol%. Diese teilneutralisierte Sprühsuspension wurde analog Beispiel 1 in einem Sprühturm sprühgetrocknet.
Die mittlere Teilchengröße des Pulvers betrug ca. 43 µm.
Das Pulver wurde analog Beispiel 1 redispergiert. Die Messung der Verteilung zeigte keine Ergebnis, da die Redispergierung völlig unzureichend war.
Die Weiterverarbeitung zu Filmtabletten war nicht möglich.

### Beispiel 8 (Vergleichsbeispiel)

3,4 ml Essigsäure wurden in 500 ml VE-Wasser gelöst und anschließend unter Rühren n 1000 ml einer wässrigen Dispersion des Polymers A mit einem Feststoffgehalt von 30 % eingearbeitet. Dies entspricht einem Neutralisationsgrad von 8 mol%. Diese teilneutralisierte Sprühsuspension wurde analog Beispiel 1 in einem Sprühturm sprühgetrocknet.
Die mittlere Teilchengröße des Pulvers betrug ca. 41 µm.
Das Pulver wurde analog Beispiel 1 redispergiert und zeigt eine monomodale Verteilung mit einem Maximum bei 191 nm.

**Tabelle 4: Zusammensetzung Kern:**

| Zuammensetzung | [%] | [mg] |
|---|---|---|
| Chininhydrochlorid-Dihydrat | 30,30 | 100,00 |
| Ludipress | 57,27 | 189,00 |
| Avicel PH 102 | 12,02 | 39,67 |
| Aerosil® 200 | 0,11 | 0,33 |
| Magnesiumstearat | 0,30 | 1,00 |
| | **100,00** | **330** |

Zur Herstellung der Kerne wurden die abgewogenen Bestandteile Chinin-HCl, Ludipress, Avicel PH 102 und Aerosil 200 in einem Diosna-Mischer für 3 Minuten auf Stufe 1 gemischt. Nach Zugabe von Magnesiumstearat wurde nochmals 1 Minute gemischt. Die so hergestellte Pulvermischung wurde auf einer Rundläuferpresse zu Tabletten mit einem Gewicht von 330 mg und einer Bruchfestigkeit von 80 N verpresst.

Die Weiterverarbeitung zu Filmtabletten mit einer Auftragsmenge von 4 mg/cm² erfolgte analog Beispiel 1. Die so hergestellten Tabletten zeigten überraschenderweise trotz sehr guter Redispergierung keine Resistenz in Puffer pH 6,8.

### Beispiel 9

6,5 g Fumarsäure wurden in 500 ml VE-Wasser gelöst und anschließend unter Rühren in 1000 ml einer wässrigen Dispersion des Polymers A mit einem Feststoffgehalt von 30 % eingearbeitet. Dies entspricht einem Neutralisationsgrad von 8 mol%. Diese teilneutralisierte Sprühsuspension wurde analog Beispiel 1 in einem Sprühturm sprühgetrocknet.
Die mittlere Teilchengröße des Pulvers betrug ca. 39 µm.
Das Pulver wurde analog Beispiel 1 redispergiert und zeigt eine monomodale Verteilung mit einem Maximum bei 183 nm.
Die Weiterverarbeitung zu Filmtabletten mit einer Auftragsmenge von 4, 8 und 12 mg/cm² erfolgte analog Beispiel 1.
Die Prüfung der Tabletten auf Resistenz in Puffer pH 6,8 zeigte folgende Ergebnisse:

| | | | |
|---|---|---|---|
| | 4 mg/cm² | 8 mg/cm² | 12 mg/cm² |
| 30 min | 100 % | 100 % | 100 % |
| 60 min | 65 % | 87 % | 100 % |

### Beispiel 10

6,5 g Fumarsäure wurden in 500 ml VE-Wasser gelöst und anschließend unter Rühren in 1000 ml einer wässrigen Dispersion des Polymers A mit einem Feststoffgehalt von 30 Gew.-% eingearbeitet. Dies entspricht einem Neutralisationsgrad von 8 mol-%. Diese teilneutralisierte Dispersion wurde in einem FSD-Sprühturm sprühgetrocknet, wobei die Zerstäubung über eine 1,4 mm Zweistoffdüse bei 0.3 MPa Zerstäubungsdruck erfolgte. Die Zulufttemperatur lag bei 110°C und die Ablufttemperatur bei 57°C. Der Feinanteil wurde während der Sprühtrocknung abgetrennt
und wieder vor die Sprühdüse geblasen, so dass sprühgetrocknete Teilchen mit einer mittleren Partikelgröße von 280 µm resultierten.
Das sprühgetrocknete Produkt wurde in Wasser zu einer Sprühsuspension mit 20 Gew.-% Feststoffgehalt durch Rühren mit einem Blattrührer für 60 min redispergiert. Die Messung der Teilchengröße mittels Lichtstreuung ergab einen Wert von 175 nm.
Die so hergestellte Zubereitung wurde mit 14 g Acetyltriethylcitrat versetzt, zwei Stunden gerührt und durch Versprühen auf Tablettenkerne (Formulierung Tabelle 1) analog Beispiel 1 aufgebracht.

Die Prüfung der Tabletten mit einer Auftragsmenge von 4 mg/cm² auf Resistenz in Puffer pH 6,8 zeigte folgende Ergebnisse

| | |
|---|---|
| 30 min | 100 % |
| 60 min | 71 % |

### Beispiel 11

6,7 g Bernsteinsäure wurden in 500 ml VE-Wasser gelöst und anschließend unter Rühren in 1000 ml einer wässrigen Dispersion des Polymers A mit einem Feststoffgehalt von 30 Gew.-% eingearbeitet. Dies entspricht einem Neutralisationsgrad von 8 mol-%. Diese teilneutralisierte Dispersion wurde analog Beispiel 10 in einem FSD-Sprühturm sprühgetrocknet. Die mittlere Teilchengröße des Pulvers betrug 312 µm.
Das sprühgetrocknete Produkt wurde in Wasser zu einer Sprühsuspension mit 20 Gew.-% Feststoffgehalt durch Rühren mit einem Blattrührer für 60 min redispergiert. Die Messung der Teilchengröße mittels Lichtstreuung ergab einen Wert von 172 nm.
Die so hergestellte Zubereitung wurde mit 14 g Acetyltriethylcitrat versetzt, zwei Stunden gerührt und durch Versprühen auf Tablettenkerne analog Beispiel 4 (Tabelle 3) aufgebracht. Die Prüfung der Tabletten mit einer Auftragsmenge von 4 mg/cm² auf Resistenz in Puffer pH 6,8 zeigte folgende Ergebnisse

| | |
|---|---|
| 30 min | 100 % |
| 60 min | 75 % |

### Beispiel 12

7,4 g Glutarsäure wurden in 500 ml VE-Wasser gelöst und anschließend unter Rühren in 1000 ml einer wässrigen Dispersion des Polymers A mit einem Feststoffgehalt von 30 Gew.-% eingearbeitet. Dies entspricht einem Neutralisationsgrad von 8 mol-%. Diese teilneutralisierte Dispersion wurde analog Beispiel 10 in einem FSD-Sprühturm sprühgetrocknet. Die mittlere Teilchengröße des Pulvers betrug 295 µm.
Das sprühgetrocknete Produkt wurde in Wasser zu einer Sprühsuspension mit 20 Gew.-% Feststoffgehalt durch Rühren mit einem Blattrührer für 60 min redispergiert. Die Messung der Teilchengröße mittels Lichtstreuung ergab einen Wert von 188 nm.
Die so hergestellte Zubereitung wurde mit 14 g Acetyltriethylcitrat versetzt, zwei Stunden gerührt und durch Versprühen auf Tablettenkerne analog Beispiel 8 (Tabelle 4) aufgebracht.
Die Prüfung der Tabletten mit einer Auftragsmenge von 4 mg/cm² auf Resistenz in Puffer pH 6,8 zeigte folgende Ergebnisse

| | |
|---|---|
| 30 min | 100 % |
| 60 min | 72 % |

### Beispiel 13

1000 ml einer wässrigen Dispersion des Polymers A mit einem Feststoffgehalt von 30 Gew.-% wurde in einem FSD-Sprühturm sprühgetrocknet, wobei die Zerstäubung über eine 1,4 mm Zweistoffdüse bei 0.3 MPa Zerstäubungsdruck erfolgte. Die Zulufttemperatur lag bei 127°C und die Ablufttemperatur bei 59°C. Der Feinanteil wurde während der Sprühtrocknung abgetrennt und wieder vor die Sprühdüse geblasen, so dass sprühgetrocknete Teilchen mit einer mittleren Partikelgröße von 220 µm resultierten.
5,2 g Fumarsäure wurden in 1000 ml VE-Wasser gelöst und anschließend 250 g des sprühgetrockneten Pulvers unter Rühren mit einem Blattrührer eingearbeitet. Dies entspricht einem Neutralisationsgrad von 8 mol-%. Nach 60 min Redispergierzeit betrug die mittlere Partikelgröße 184 nm.
Die so hergestellte Zubereitung wurde mit 35 g Acetyltriethylcitrat versetzt, zwei Stunden gerührt und durch Versprühen auf Tablettenkerne (Formulierung Tabelle 3) analog Beispiel 1 aufgebracht.
Die Prüfung der Tabletten mit einer Auftragsmenge von 4 mg/cm² auf Resistenz in Puffer pH 6,8 zeigte folgende Ergebnisse

| | |
|---|---|
| 30 min | 100 % |
| 60 min | 68 % |

### Beispiel 14

5,3 g Bernsteinsäure wurden in 1000 ml VE-Wasser gelöst und anschließend 250 g des sprühgetrockneten Pulvers aus Beispiel 13 unter Rühren mit einem Blattrührer eingearbeitet. Dies entspricht einem Neutralisationsgrad von 8 mol -%. Nach 60 min Redispergierzeit betrug die mittlere Partikelgröße 165 nm.
Die so hergestellte Zubereitung wurde mit 35 g Acetyltriethylcitrat versetzt, zwei Stunden gerührt und durch Versprühen auf Tablettenkerne (Formulierung Tabelle 4) analog Beispiel 1 aufgebracht.
Die Prüfung der Tabletten mit einer Auftragsmenge von 4 mg/cm² auf Resistenz in Puffer pH 6,8 zeigte folgende Ergebnisse

| | |
|---|---|
| 30 min | 100 % |
| 60 min | 79 % |

### Beispiel 15

5,9 g Glutarsäure wurden in 1000 ml VE-Wasser gelöst und anschließend 250 g des sprühgetrockneten Pulvers aus Beispiel 13 unter Rühren mit einem Blattrührer eingearbeitet. Dies entspricht einem Neutralisationsgrad von 8 mol-%. Nach 60 min Redispergierzeit betrug die mittlere Partikelgröße 174 nm.

Die so hergestellte Zubereitung wurde mit 35 g Acetyltriethylcitrat versetzt, zwei Stunden gerührt und durch Versprühen auf Tablettenkerne (Formulierung Tabelle 1) analog Beispiel 1 aufgebracht.
Die Prüfung der Tabletten mit einer Auftragsmenge von 4 mg/cm² auf Resistenz in Puffer pH 6,8 zeigte folgende Ergebnisse

| | |
|---|---|
| 30 min | 100 % |
| 60 min | 69 % |

### Beispiel 16

6,5 g Fumarsäure wurden in 500 ml VE-Wasser gelöst und anschließend unter Rühren in 1000 ml einer wässrigen Dispersion des Polymers A mit einem Feststoffgehalt von 30 Gew.-% eingearbeitet. Dies entspricht einem Neutralisationsgrad von 8mol%. Diese teilneutralisierte Dispersion wurde analog Beispiel 10 in einem FSD-Sprühturm sprühgetrocknet. Die mittlere Teilchengröße des Pulvers betrug 280 µm.
Das sprühgetrocknete Produkt wurde in Wasser zu einer Sprühsuspension mit 20 Gew.-% Feststoffgehalt durch Rühren mit einem Blattrührer für 60 min redispergiert. Die Messung der Teilchengröße mittels Lichtstreuung ergab einen Wert von 175 nm.
Die so hergestellte Zubereitung wurde mit 14 g Acetyltriethylcitrat versetzt, zwei Stunden gerührt und durch Versprühen analog Beispiel 1 auf Coffeinpellets aufgebracht.

**Tabelle 5 : Zusammensetzung Pellets:**

| Zuammensetzung | [Gew.-%] |
|---|---|
| Coffein | 20,0 |
| Laktose | 38,5 |
| Mikrokristalline Cellulose | 38,5 |
| Kollidon® CL-F | 3,0 |
| | **100,00** |

Die Bestandteile wurden in einem Diosna-Mischer für 3 min gemischt und anschließend mit Wasser befeuchtet. Diese feuchte Masse wurde extrudiert und anschließend in einer Rundungsmaschine zu Pellets mit einem Durchmesser von 0,7 - 1,4 mm ausgerundet.

Die Prüfung der Pellets mit einer Auftragsmenge von 4 mg/cm² auf Resistenz in Puffer pH 6,8 zeigte folgende Ergebnisse

| | |
|---|---|
| 30 min | 100 % |
| 60 min | 87 % |

### Beispiel 17

6,7 g Bernsteinsäure wurden in 500 ml VE-Wasser gelöst und anschließend unter Rühren in 1000 ml einer wässrigen Dispersion des Polymers A mit einem Feststoffgehalt von 30 Gew.-% eingearbeitet. Dies entspricht einem Neutralisationsgrad von 8mol%. Diese teilneutralisierte Dispersion wurde analog Beispiel 10 in einem FSD-Sprühturm sprühgetrocknet. Die mittlere Teilchengröße des Pulvers betrug 312 µm.
Das sprühgetrocknete Produkt wurde in Wasser zu einer Sprühsuspension mit 20 Gew.-% Feststoffgehalt durch Rühren mit einem Blattrührer für 60 min redispergiert. Die Messung der Teilchengröße mittels Lichtstreuung ergab einen Wert von 172 nm.
Die so hergestellte Zubereitung wurde mit 14 g Acetyltriethylcitrat versetzt, zwei Stunden gerührt und durch Versprühen auf Theophyllin-Granulat (Teilchengröße von 0,2 - 0,7 mm) analog Beispiel 1 aufgebracht.
Die Prüfung des Granulats mit einer Auftragsmenge von 4 mg/cm² auf Resistenz in Puffer pH 6,8 zeigte folgende Ergebnisse:

| | |
|---|---|
| 30 min | 100 % |
| 60 min | 91 % |

### Beispiel 18

7,4 g Glutarsäure wurden in 500 ml VE-Wasser gelöst und anschließend unter Rühren in 1000 ml einer wässrigen Dispersion des Polymers A mit einem Feststoffgehalt von 30 Gew.-% eingearbeitet. Dies entspricht einem Neutralisationsgrad von 8 mol-%. Diese teilneutralisierte Dispersion wurde analog Beispiel 10 in einem FSD-Sprühturm sprühgetrocknet. Die mittlere Teilchengröße des Pulvers betrug 295 µm.
Das sprühgetrocknete Produkt wurde in Wasser zu einer Sprühsuspension mit 20 Gew.-% Feststoffgehalt durch Rühren mit einem Blattrührer für 60 min redispergiert. Die Messung der Teilchengröße mittels Lichtstreuung ergab einen Wert von 188 nm.
Die so hergestellte Zubereitung wurde mit 14 g Acetyltriethylcitrat versetzt, zwei Stunden gerührt und analog Beispiel 1 durch Versprühen auf Paracetamol-Kristalle (Durchmesser 0,3mm) aufgebracht.
Die Prüfung des Granulats mit einer Auftragsmenge von 4 mg/cm² auf Resistenz in Puffer pH 6,8 zeigte folgende Ergebnisse:

| | |
|---|---|
| 30 min | 100 % |
| 60 min | 90 % |

### Beispiel 19

27,6 g Natriumlaurylsulfat und 14,7 g Fumarsäure wurden in 1500 ml VE-Wasser gelöst und anschließend unter Rühren in 3000 ml einer wässrigen Dispersion des Polymers A mit einem Feststoffgehalt von 30 Gew.-% eingearbeitet. Dies entspricht einem Neutralisationsgrad von 6 mol-%. Diese teilneutralisierte Sprühsuspension wurde in einem Sprühturm sprühgetrocknet.

Die Zerstäubung erfolgte hierbei über eine 1,4 mm Zweistoffdüse mit 0.3 MPa Zerstäubungsdruck. Das Trocknungsgas wurde tangential im Eingangsbereich des Sprühtrockners zugeführt und das getrocknete Produkt in einem Zyklon abgeschieden. Die Zulufttemperatur betrug 112°C und die Ablufttemperatur 59°C. Die mittlere Teilchengröße des Pulvers betrug 37 µm.
750 g des sprühgetrockneten Pulvers wurde in VE-Wasser zu einer Sprühsuspension mit 20 Gew.-% Feststoffgehalt durch Rühren 60 Minuten mit einem Propellerrührer redispergiert. Die Messung der Teilchengröße mittels Lichtstreuung zeigte eine monomodale Verteilung mit einem Maximum bei 173 nm. Die Sprühsuspension wurde mit 13 Gew.-% Tributylcitrat bezogen auf Feststoff Polymer versetzt und nach zwei Stunden Rühren mit den in Tabelle 5 aufgelisteten Parametern weiterverarbeitet.

**Tabelle 5: Sprühbedingungen:**

| Equipment | Manesty |
|---|---|
| Zulufttemperatur | 60°C |
| Zuluftmenge | 450m³/h |
| Trommeldrehzahl | 14U/min |
| Ansatzgröße | 5kg |
| Kern | Beispiel 1 |
| Sprührate | 25g/min |
| Zerstäubungsdruck | 0.28 MPa |
| Sprühbreite | 0.28 MPa |
| Auftragsmenge | 4mg/cm² |

Die Prüfung der Tabletten auf Resistenz in Puffer pH 6,8 zeigte folgende Ergebnisse:

| | |
|---|---|
| 30 min | 100 % |
| 60 min | 69 % |

### Beispiel 20

### Pulver A:

1000 ml einer wässrigen Dispersion des Polymers A mit einem Feststoffgehalt von 30 Gew.-% wurden in einem Sprühturm sprühgetrocknet. Die Zerstäubung erfolgte hierbei über eine 1,4 mm Zweistoffdüse mit 0.3 MPa Zerstäubungsdruck. Das Trocknungsgas wurde tangential im Eingangsbereich des Sprühtrockners zugeführt und das getrocknete Produkt in einem Zyklon abgeschieden. Die Zulufttemperatur betrug 109°C und die Ablufttemperatur 58°C. Die mittlere Teilchengröße des Pulvers betrug 33 µm.

### Pulver B:

105,1 g Weinsäure wurden in 500 ml VE-Wasser gelöst und anschließend unter Rühren in 1000 ml einer wässrigen Dispersion des Polymers mit einem Feststoffgehalt von 30 Gew.-% eingearbeitet. Dies entspricht einem Neutralisationsgrad von 100 mol-%. Diese Sprühsuspension wurde in einem Sprühturm analog Pulver A sprühgetrocknet.
Die mittlere Teilchengröße des Pulvers betrug 35 µm.
Pulver A wurde mit Pulver B im Turbula gemischt, so dass sich ein Neutralisationsgrad von 7 mol-% einstellte und anschließend in VE-Wasser unter Rühren mittels Blattrührer zu einer 20 % gew.-igen Dispersion redispergiert.
Die Messung der Teilchengröße mittels Lichtstreuung zeigte eine monomodale Verteilung mit einem Maximum bei 168 nm. Nach Zusetzen von 13 Gew.-% Acetyltriethylcitrat bezogen auf Polymer wurde die Sprühsuspension analog Beispiel 19 auf Coffeinkerne gemäß Tabelle 1 aufgesprüht.
Die Prüfung der Tabletten mit einer Auftragsmenge von 4 mg/cm² auf Resistenz in Puffer pH 6,8 zeigte folgende Ergebnisse

| | |
|---|---|
| 30 min | 100 % |
| 60 min | 72 % |

### Beispiel 21

14,2 g Sebacinsäure wurden in 500 ml VE-Wasser suspendiert und anschließend unter Rühren in 100 ml einer wässrigen Dispersion des Polymers A mit einem Feststoffgehalt von 30 Gew.-% eingearbeitet. Dies entspricht einem Neutralisationsgrad von 100 mol-%.
Diese Lösung wurde unter Rühren in 1300 ml einer wässrigen Dispersion des Polymers mit einem Feststoffgehalt von 30 Gew.-% eingearbeitet, so dass sich ein Neutralisationsgrad von 7 mol-% einstellte. Die so hergestellte Sprühsuspension wurde in einem FSD-Sprühturm sprühgetrocknet, wobei die Zerstäubung über eine 1,4 mm Zweistoffdüse bei 0.3 MPa Zerstäubungsdruck erfolgte. Die Zulufttemperatur lag bei 118°C und die Ablufttemperatur bei 63°C. Der Feinanteil wurde während der Sprühtrocknung abgetrennt und wieder vor die Sprühdüse geblasen, so dass sprühgetrocknete Teilchen mit einer mittleren Partikelgröße von 263 µm resultierten. 100 g des sprühgetrockneten Produkts wurde in Wasser zu einer Sprühsuspension mit 20 Gew.-% Feststoffgehalt durch Rühren mit einem Blattrührer für 60 min dispergiert. Die Messung der Teilchengröße mittels Lichtstreuung zeigte eine monomodale Verteilung mit einem Maximum bei 178 nm.
Die so hergestellte Zubereitung wurde mit 15 Gew.-% Acetyltriethylcitrat bezogen auf Feststoff Polymer versetzt, zwei Stunden gerührt und analog Beispiel 19 durch Versprühen auf Tablettenkerne gemäß Tabelle 4 aufgebracht.
Die Prüfung der Tabletten mit einer Auftragsmenge von 4 mg /cm² auf Resistenz in Puffer pH 6,8 zeigte folgende Ergebnisse:

| | |
|---|---|
| 30 min | 100 % |
| 60 min | 78 % |

### Beispiel 22

50 g PEG 6000, 200 g Talkum, 9 g Titandioxid und 9 g Eisenoxid rot wurden in einem Turbulamischer vermischt und anschließend in einer Stiftmühle gemahlen. Diese Mischung wurde in 250 g VE-Wasser suspendiert und auf 500 g Polymerpulver hergestellt nach Beispiel 10 mittels Diosna-Granulation aufgebracht, so dass Granulate mit einer mittleren Partikelgröße von 187 µm resultieren.

**Tabelle 6: Granulierbedingungen:**

| Equipment | Diosna |
|---|---|
| Mischerdrehzahl | 350 Upm |
| Zerhackerdrehzahl | 2000 Upm |

100 g dieser Granulate wurde in Wasser zu einer Sprühsuspension mit 20 Gew.-% Feststoffgehalt durch Rühren mit einem Blattrührer für 60 min dispergiert. Die Messung der Teilchengröße mittels Lichtstreuung zeigte eine monomodale Verteilung mit einem Maximum bei 177 nm.
Die so hergestellte Zubereitung wurde mit 15 % Gew.-Acetyltriethylcitrat bezogen auf Feststoff Polymer versetzt, zwei Stunden gerührt und analog Beispiel 1 durch Versprühen auf Tablettenkerne gemäß Tabelle 3 aufgebracht.

Die Prüfung der Tabletten mit einer Auftragsmenge von 4 mg /cm² auf Resistenz in Puffer pH 6,8 zeigte folgende Ergebnisse:

| | |
|---|---|
| 30 min | 100 % |
| 60 min | 82 % |

### Beispiel 23

500 g Polymerpulver hergestellt nach Beispiel 11 wurden mit 3 g Lecithin, 6 g Stearinsäure und 150 g Talkum in einem Turbulamischer vermischt und anschließend mittels Kompaktor weiterverarbeitet. Die so entstandenen Schuppen wurden gemahlen und anschließend in VE-Wasser redispergiert. Die Messung der Teilchengröße mittels Lichtstreuung zeigte eine monomodale Verteilung mit einem Maximum bei 181 nm.
2,5 Gew.-% BHT wurden in 15 Gew.-% Acetyltriethylcitrat (bezogen auf Feststoff Polymer) gelöst und anschließend zur Dispersion gegeben.
Die so hergestellte Zubereitung wurde mit 15 Gew.-% Acetyltriethylcitrat bezogen auf Feststoff Polymer versetzt, zwei Stunden gerührt und analog Beispiel 1 durch Versprühen auf Tablettenkerne gemäß Tabelle 1 aufgebracht.
Die Prüfung der Tabletten mit einer Auftragsmenge von 4 mg /cm² auf Resistenz in Puffer pH 6,8 zeigte folgende Ergebnisse.

| | |
|---|---|
| 30 min | 100 % |
| 60 min | 84 % |

### Beispiel 24

200 g Talkum und 8 g Indigotinlack wurden in 500 g VE-Wasser suspendiert und anschließend auf 500 g Polymerpulver hergestellt nach Beispiel 12 in einem Wirbelschichtverfahren aufgebracht.

**Tabelle 7 : Granulierbedingungen:**

| Equipment | Glatt GPC 1 |
|---|---|
| Zulufttemperatur | 60°C |
| Zuluftmenge | 300 m³/h |
| Ansatzgröße | 500g |
| Sprühdruck | 0.15 MPa |

Die mittlere Teilchengröße des Granulats betrug ca. 312 µm.
100 g des Granulats wurde in VE-Wasser zu einer Sprühsuspension mit 20 Gew.-% Feststoffgehalt durch Rühren 60 min mit einem Propellerrührer redispergiert. Die Messung der Teilchengröße mittels Lichtstreuung zeigte eine monomodale Verteilung mit einem Maximum bei 193 nm. Die Sprühsuspension wurde mit 13 Gew.-% Tributylcitrat bzg. auf Feststoff Polymer versetzt und nach zwei Stunden rühren analog Beispiel 19 durch Versprühen auf Tablettenkerne gemäß Tabelle 4 weiterverarbeitet.
Bei einer Auftragsmenge von 4 mg/cm² zeigten 74% der gecoateten Tabletten nach 30 min eine Resistenz von 100 %.

### Beispiel 25

200 g Talkum, 8 g Indigotinlack sowie 11,9 g Glutarsäure wurden in 500 g VE-Wasser suspendiert und anschließend auf 500 g Polymerpulver hergestellt nach Beispiel 13 in einem Wirbelschichtverfahren analog Beispiel 11 aufgebracht. Dies entspricht einem Neutralisationsgrad von 8 mol-%.
Die mittlere Teilchengröße des Granulats betrug 298 µm.
100 g des Granulats wurde in VE-Wasser zu einer Sprühsuspension mit 20 Gew.-% Feststoffgehalt durch Rühren 60 min mit einem Propellerrührer redispergiert. Die Messung der Teilchengröße mittels Lichtstreuung zeigte eine monomodale Verteilung mit einem Maximum bei 184 nm. Die Sprühsuspension wurde mit 13 Gew.-% Tributylcitrat, bezogen auf Feststoff Polymer, versetzt und nach zwei Stunden rühren analog Beispiel 1 durch Versprühen auf Tablettenkerne gemäß Tabelle 3 weiterverarbeitet.
Bei einer Auftragsmenge von 4 mg/cm² zeigten die gecoateten Tabletten nach 60 min eine Resistenz von 83 %.

### Beispiel 26

100 g Talkum, 20 g Eisenoxid rot sowie 5,2 g Malonsäure wurden in 300 g VE-Wasser suspendiert und anschließend mittels eines Ultra-Turrax 15 min bei 10 000 U/min homogenisiert. Diese Pigmentsuspension wurde unter Rühren in 1000 ml einer wässrigen Dispersion des Polymers A mit einem Feststoffgehalt von 30 Gew.-% eingearbeitet. Die so hergestellte Sprühsuspension mit einem Neutralisationsgrad von 7 mol% wurde anschließend in einem SBD-Sprühturm sprühgetrocknet, wobei die Zerstäubung über eine 1,4 mm Zweistoffdüse bei 0.30 MPa Zerstäubungsdruck erfolgte. Die Zulufttemperatur betrug 135°C und die Ablufttemperatur bei 63°C. Der Feinanteil wurde während der Sprühtrocknung abgetrennt und wieder vor die Sprühdüse geblasen, so dass sprühgetrocknete Teilchen mit einer mittleren Partikelgröße von 320 µm resultierten.
Das sprühgetrocknete Produkt wurde in Wasser zu einer Sprühsuspension mit einem Feststoffgehalt von 20 Gew.-% durch Rühren mit einem Blattrührer eingearbeitet. Die so hergestellte Zubereitung wurde mit 15 % Triethylcitrat bzg. auf Feststoff Polymer versetzt, weitere zwei Stunden gerührt und durch Versprühen auf Tablettenkerne (Tabelle 1) analog Beispiel 1 aufgebracht.
Die gecoateten Tabletten mit einer Auftragsmenge von 4 mg/cm² zeigten nach 60 min in Puffer pH 6,8 eine Resistenz von 100 %.

### Beispiel 27

100 g Polymerpulver hergestellt nach Beispiel 10 wurden mit 50 g Talkum und 4 g Indigotinlack in einem Turbulamischer vermischt.
Nach der Redispergierung dieser Zubereitung in Wasser zu einer 20 % gew.-igen Suspension mittels eines Blattrührers ergab eine Teilchengröße von 182 nm. Die Suspension wurde mit 15 Gew.-% Dibutylsebacat versetzt und nach zwei Stunden Rühren analog Beispiel 1 auf Tabletten gemäß Tabelle 4 aufgesprüht. Bei einer Auftragsmenge von 4 mg/cm² zeigten die gecoateten Tabletten nach 30 min eine Resistenz von 100 % und nach 60 min eine Resistenz von 78 %.

### Beispiel 28

100 g Polymerpulver hergestellt nach Beispiel 13 wurden mit 50 g feinst gemahlenem Talkum, 4 g Indigotinlack und 2,3 g Sebacinsäure in einem Turbulamischer vermischt.
Nach der Redispergierung dieser Zubereitung in Wasser zu einer 20 % gew.-igen Suspension mit einem Neutralisationsgrad von 5 mol-% mittels eines Blattrührers betrug die Teilchengröße 175 nm. Die Suspension wurde mit 15 Gew.-% Dibutylsebacat und 0,5 % Simethicon® versetzt und nach zwei Stunden Rühren analog Beispiel 1 auf Tabletten gemäß Tabelle 4 aufgesprüht. Bei einer Auftragsmenge von 4 mg/cm² zeigten die gecoateten Tabletten nach 30 min eine Resistenz von 100 %.

### Beispiel 29

100 g Polymerpulver hergestellt nach Beispiel 11 wurden mit 60 g Talkum, 6 g Eisenoxid rot und 0,5 g Lecithin in einem Turbulamischer vermischt und anschließend in einer Stiftmühle vermahlen.
Nach der Redispergierung dieser Zubereitung in Wasser zu einer 20 % gew.-igen Suspension mittels eines Blattrührers ergab sich eine Teilchengröße von 176 nm, wobei für diese Bestimmung Talkum und Eisenoxid vorher abzentrifugiert wurden.
Die Suspension wurde mit 15 Gew.-% Dibutylsebacat versetzt und nach zwei Stunden Rühren analog Beispiel 19 auf Tabletten gemäß Tabelle 1 aufgesprüht. Bei einer Auftragsmenge von 4 mg/cm² zeigten die gecoateten Tabletten nach 60 min eine Resistenz von 83 %.

### Beispiel 30

1000 ml einer wässrigen Dispersion des Polymers A mit einem Feststoffgehalt von 30 Gew.-% wurden in einem SBD-Sprühturm sprühgetrocknet, wobei die Zerstäubung über eine 1,4 mm Zweistoffdüse bei 0.3 MPa Zerstäubungsdruck erfolgte. Die Zulufttemperatur lag bei 110°C und die Ablufttemperatur bei 57°C. Der Feinanteil wurde während der Sprühtrocknung abgetrennt und wieder vor die Sprühdüse geblasen, so dass sprühgetrocknete Teilchen mit einer mittleren Partikelgröße von 190 µm resultierten. 30 g Titandioxid, 200 g Talkum, 8,4 g Adipinsäure und 25 g Eisenoxid wurden in einer Stiftmühle gemahlen, anschließend mit dem sprühgetrockneten Pulver vermischt und in VE-Wasser zu einer Sprühsuspension mit 20 Gew.-% Feststoffgehalt durch Rühren mit einem Blattrührer für 60 min redispergiert. Die Messung der Teilchengröße mittels Lichtstreuung ergab einen Wert von 170 nm.
Die so hergestellte Zubereitung wurde mit 15 Gew.-% Triethylcitrat, bezogen auf Feststoff Polymer, versetzt, zwei Stunden gerührt und analog Beispiel 19 durch Versprühen auf Tablettenkerne gemäß Tabelle 1 aufgebracht.
Die Prüfung der Tabletten auf Resistenz in Puffer pH 6,8 zeigte folgende Ergebnisse:

| | |
|---|---|
| 30 min | 100 % |
| 60 min | 76 % |

### Beispiel 31

1000 ml einer wässrigen Dispersion des Polymers A mit einem Feststoffgehalt von 30 Gew.-% wurden in einem Sprühturm sprühgetrocknet, wobei die Zerstäubung über eine 1,4 mm Zweistoffdüse bei 0.3 MPa Zerstäubungsdruck erfolgte. Die Zulufttemperatur lag bei 112°C und die Ablufttemperatur bei 58°C.
Dieses Pulver wurde mit 8,4 g fein gepulverter Adipinsäure, 60 g feinem Talkum, 2 g feinem Xanthan, 15 g feinem Eisenoxid rot vermischt und anschließend unter Rühren in Wasser zu einer Sprühsuspension mit 20 % Feststoffgehalt durch Rühren mit einem Blattrührer für 60 min redispergiert. Die Messung der Teilchengröße mittels Lichtstreuung ergab einen Wert von 168 nm, wobei für diese Bestimmung Talkum und Eisenoxid vorher abzentrifugiert wurden.

Die so hergestellte Zubereitung wurde mit 15 Gew.-% Triethylcitrat, bezogen auf Feststoff Polymer, versetzt, zwei Stunden gerührt und analog Beispiel 19 durch Versprühen auf Tablettenkerne gemäß Tabelle 1 aufgebracht.
Die Prüfung der Tabletten auf Resistenz in Puffer pH 6,8 zeigte folgende Ergebnisse.

| | |
|---|---|
| 30 min | 100 % |
| 60 min | 79 % |

### Beispiel 32

1000 ml einer wässrigen Dispersion des Polymers A mit einem Feststoffgehalt von 30 Gew.-% wurde in einem FSD-Sprühturm sprühgetrocknet, wobei die Zerstäubung über eine 1,4 mm Zweistoffdüse bei 0.3 MPa Zerstäubungsdruck erfolgte. Die Zulufttemperatur lag bei 123°C und die Ablufttemperatur bei 54°C. Der Feinanteil wurde während der Sprühtrocknung abgetrennt und wieder vor die Sprühdüse geblasen, so dass sprühgetrocknete Teilchen mit einer mittleren Partikelgröße von 211 µm resultierten.
15,1 g / 37,7 g / 75,5 g Äpfelsäure wurden jeweils in 1000 ml VE-Wasser gelöst und anschließend 250 g des sprühgetrockneten Pulvers unter Rühren mit einem Blattrührer in die jeweilige Äpfelsäurelösung eingearbeitet. Dies entspricht einem Neutralisationsgrad von 2, 5 bzw. 10 mol-%. Nach 60 min Redispergierzeit betrug die mittlere Partikelgröße der jeweiligen Disperionen 220/198/182 nm.
Die so hergestellte Zubereitung wurde mit 35 g Acetyltriethylcitrat versetzt, zwei Stunden gerührt und analog Beispiel 1 durch Versprühen auf Tablettenkerne gemäß Tabelle 3 aufgebracht. Die Prüfung der Tabletten mit einer Auftragsmenge von 4 mg/cm² auf Resistenz in Puffer pH 6,8 zeigte folgende Ergebnisse.

| | | | |
|---|---|---|---|
| | 2 mol-% | 5 mol-% | 10 mol-% |
| 30 min | 100 % | 100 % | 100 % |
| 60 min | 37 % | 65 % | 89 % |

### Beispiel 33

1,3 g Bernsteinsäure, 1,2 g Fumarsäure und 1,6 g Weinsäure wurden jeweils in 150 ml VE-Wasser gelöst und anschließend unter Rühren in 1000 ml einer wässrigen Dispersion des Polymers A mit einem Feststoffgehalt von 30 Gew.-% eingearbeitet. Dies entspricht einem Neutralisationsgrad von jeweils 1,5 mol-%. Diese teilneutralisierte Sprühsuspension wurde analog Beispiel 1 in einem Sprühturm sprühgetrocknet.
Die mittlere Teilchengröße des Pulvers betrug 37 µm.
Das Pulver wurde analog Beispiel 1 redispergiert und zeigt eine monomodale Verteilung mit einem Maximum bei 182 nm.
Die Weiterverarbeitung zu Filmtabletten mit einer Auftragsmenge von 4 mg/cm² erfolgte analog Beispiel 1.

Die Prüfung der Tabletten auf Resistenz in Puffer pH 6,8 zeigte folgende Ergebnisse:

| | |
|---|---|
| 30 min | 100 % |
| 60 min | 72 % |

## Patentansprüche

1. Pharmazeutische Überzüge erhalten aus filmbildenden Überzugsmitteln auf Basis von Copolymeren aus N,N-Diethylaminoethylmethacrylat und Methylmethacrylat im Gewichtsverhältnis der Monomeren von 35:65 bis 55:45, wobei die Copolymeren zu 2 bis 15 mol-% mit C₃-C₁₀-Dicarbonsäuren teilneutralisiert vorliegen.

2. Überzüge nach Anspruch 1, wobei die Überzugsmittel in Auftragsmengen von 1 bis 20 mg/cm² aufgetragen werden und die Überzüge bei Auftragsmengen von 4 mg/cm² eine Resistenz gegen Freisetzung des Wirkstoffs im wässrigen Milieu bei pH-Wert 6,8 von mindestens 80 % nach 30 min aufweisen.

3. Überzüge nach Anspruch 1 oder 2, wobei die Auftragsmenge des Überzugsmittels von 2 bis 15 mg/cm beträgt.

4. Überzüge nach einem der Ansprüche 1 bis 3, wobei mit einer Auftragsmenge des Überzugsmittels von 4 bis 12 mg/cm².

5. Überzüge nach einem der Ansprüche 1 bis 4, wobei die Copolymeren mit unverzweigten C₃-C₁₀-Dicarbonsäuren teilneutralisiert vorliegen.

6. Überzüge nach einem der Ansprüche 1 bis 5, wobei die Dicarbonsäure Fumarsäure ist.

7. Überzüge nach einem der Ansprüche 1 bis 6, wobei die Dicarbonsäuren Alkandicarbonsäuren sind.

8. Überzüge nach einem der Ansprüche 1 bis 7, wobei die Dicarbonsäuren ausgewählt sind aus der Gruppe bestehend aus Malonsäure, Bernsteinsäure, Glutarsäure, Adipinsäure oder Sebacinsäure. Äpfelsäure (2-Hydroxy-Bernsteinsäure) und Weinsäure (2,3-Dihydroxybernsteinsäure).

9. Überzüge nach einem der Ansprüche 1 bis 8, wobei die Copolymere zu 4 bis 10 mol-% teilneutralisiert vorliegen.

10. Überzüge nach einem der Ansprüche 1 bis 9, enthaltend Dicarbonsäuren, die einen ersten pKₛ-Wert von größer 2 und einen zweiten pKₛ-Wert von größer 4 aufweisen.

11. Überzüge nach Anspruch 10, enthaltend als Dicarbonsäure Adipinsäure.

12. Überzüge nach einem der Ansprüche 1 bis11, erhalten durch Aufbringen eines wässrigen Überzugsmittels, welches durch Redispergierung des Überzugsmittels aus der Pulverform hergestellt wird.

13. Verfahren zur Herstellung von Überzugsmitteln für Überzüge von mindestens einen pharmazeutischen Wirkstoff enthaltende Dosierungsformen mit einer Resistenz gegen vorzeitige Freisetzung des Wirkstoffs, wobei die Überzugsmittel als Polymere Copolymere aus N,N-Diethylaminoethylmethacrylat und Methylmethacrylat im Gewichtsverhältnis der Monomeren von 35:65 bis 55:45 enthalten, **dadurch gekennzeichnet, dass** die Copolymere im Überzugsmittel zu 2 bis 15 mol-%mit C₃-C₁₀- Dicarbonsäuren teilneutralisiert werden.

14. Verfahren nach Anspruch13, **dadurch gekennzeichnet, dass** die filmbildenden Copolymere mit den Dicarbonsäuren in wässriger Dispersion teilneutralisiert werden.

15. Verfahren nach Anspruch14, **dadurch gekennzeichnet, dass** die filmbildenden Copolymere in Pulverform teilneutralisiert werden.

16. Verfahren nach einem der Ansprüche 13 bis15, **dadurch gekennzeichnet, dass** die filmbildenden Copolymere zu 4 bis 10 mol-% teilneutralisiert vorliegen.

17. Verfahren nach einem der Ansprüche 13 bis16, **dadurch gekennzeichnet, dass** die Copolymeren mit unverzweigten C₃-C₁₀-Dicarbonsäuren teilneutralisiert werden.

18. Verfahren nach einem der Ansprüche 13 bis17, **dadurch gekennzeichnet, dass** als Dicarbonsäuren Alkandicarbonsäuren werden.

19. Verfahren nach einem der Ansprüche 13 bis18, **dadurch gekennzeichnet, dass** Dicarbonsäuren ausgewählt aus der Gruppe bestehend aus Malonsäure, Bernsteinsäure, Glutarsäure, Adipinsäure oder Sebacinsäure. Äpfelsäure (2-Hydroxy-Bernsteinsäure) und Weinsäure (2,3-Dihydroxybernsteinsäure) eingesetzt werden.

20. Verfahren nach einem der Ansprüche 13 bis19, **dadurch gekennzeichnet, dass** als Dicarbonsäure Adipinsäure eingesetzt wird.

21. Verfahren nach einem der Ansprüche 13 bis20, **dadurch gekennzeichnet, dass** als Dicarbonsäure Fumarsäure eingesetzt wird.

22. Verfahren nach einem der Ansprüche 14 bis 23, **dadurch gekennzeichnet, dass** die pulverförmigen Überzugsmittel vor dem Auftragen auf die Dosierungsform in Wasser redispergiert werden.

23. Verwendung von Überzugsmitteln nach einem der Ansprüche 14 bis 24 für pharmazeutische Dosierungsformen auf Basis von Copolymeren aus N,N-Diethylaminoethylmethacrylat und Methylmethacrylat im Gewichtsverhältnis der Monomeren von 35:65 bis 55:45, wobei die Copolymeren zu 2 bis 15 mol-% mit C₃-C₁₀-Dicarbonsäuren teilneutralisiert vorliegen, zum Schutz von wirkstoffhaltigen pharmazeutischen Dosierungsformen gegen vorzeitige Wirkstofffreisetzung im wässrigen Milieu bei pH 6,8.

## Claims

1. A pharmaceutical coating obtained from film-forming coating compositions based on copolymers of N,N-diethylaminoethyl methacrylate and methyl methacrylate in the weight ratio of the monomers of 35:65 to 55:45, where the copolymers are present partially neutralized to 2 to 15 mol% with C₃-C₁₀-dicarboxylic acids.

2. The coating according to claim 1, where the coating compositions are applied in application amounts of from 1 to 20 mg/cm² and the coatings, for application amounts of 4 mg/cm², have a resistance to release of the active ingredient in the aqueous environment at pH 6.8 of at least 80% after 30 min.

3. The coating according to claim 1 or 2, where the application amount of the coating composition is from 2 to 15 mg/cm.

4. The coating according to any one of claims 1 to 3, where with an application amount of the coating composition of from 4 to 12 mg/cm².

5. The coating according to any one of claims 1 to 4, where the copolymers are present partially neutralized with unbranched C₃-C₁₀-dicarboxylic acids.

6. The coating according to any one of claims 1 to 5, where the dicarboxylic acid is fumaric acid.

7. The coating according to any one of claims 1 to 6, where the dicarboxylic acids are alkanedicarboxylic acids.

8. The coating according to any one of claims 1 to 7, where the dicarboxylic acids are selected from the group consisting of malonic acid, succinic acid, glutaric acid, adipic acid or sebacic acid, malic acid (2-hydroxysuccinic acid) and tartaric acid (2,3-dihydroxysuccinic acid).

9. The coating according to any one of claims 1 to 8, where the copolymers are present partially neutralized to 4 to 10 mol%.

10. The coating according to any one of claims 1 to 9, comprising dicarboxylic acids which have a first pKₐ value of greater than 2 and a second pKₐ value of greater than 4.

11. The coating according to claim 10, comprising adipic acid as dicarboxylic acid.

12. The coating according to any one of claims 1 to 11, obtained by applying an aqueous coating composition which is prepared by redispersing the coating composition from the powder form.

13. A method for producing coating compositions for coatings of dosage forms comprising at least one pharmaceutical active ingredient and having a resistance to premature release of the active ingredient, where the coating compositions comprise, as polymers, copolymers of N,N-diethylaminoethyl methacrylate and methyl methacrylate in the weight ratio of the monomers of 35:65 to 55:45, wherein the copolymers in the coating composition are partially neutralized to 2 to 15 mol% with C₃-C₁₀-dicarboxylic acids.

14. The method according to claim 13, wherein the film-forming polymers are partially neutralized with the dicarboxylic acids in aqueous dispersion.

15. The method according to claim 14, wherein the film-forming copolymers are partially neutralized in powder form.

16. The method according to any one of claims 13 to 15, wherein the film-forming copolymers are present partially neutralized to 4 to 10 mol%.

17. The method according to any one of claims 13 to 16, wherein the copolymers are partially neutralized with unbranched C₃-C₁₀-dicarboxylic acids.

18. The method according to any one of claims 13 to 17, wherein as the dicarboxylic acids are alkanedicarboxylic acids.

19. The method according to any one of claims 13 to 18, wherein dicarboxylic acids selected from the group consisting of malonic acid, succcinic acid, glutaric acid, adipic acid or sebacic acid, malic acid (2-hydroxysuccinic acid) and tartaric acid (2,3-dihydroxysuccinic acid) are used.

20. The method according to any one of claims 13 to 19, wherein the dicarboxylic acid used is adipic acid.

21. The method according to any one of claims 13 to 20, wherein the dicarboxylic acid used is fumaric acid.

22. The method according to any one of claims 14 to 23, wherein the pulverulent coating compositions are redispersed in water prior to application to the dosage form.

23. The use of coating compositions according to any one of claims 14 to 24 for pharmaceutical dosage forms based on copolymers of N,N-diethylaminoethyl methacrylate and methyl methacrylate in the weight ratio of the monomers of 35:65 to 55:45, where the copolymers are present partially neutralized to 2 to 15 mol% with C₃-C₁₀-dicarboxylic acids, for protecting active-ingredient-containing pharmaceutical dosage forms against premature active ingredient release in an aqueous environment at pH 6.8.

## Revendications

1. Revêtements pharmaceutiques obtenus à partir d'agents de revêtement filmogènes à base de copolymères de méthacrylate de N,N-diéthylaminoéthyle et de méthacrylate de méthyle en un rapport en poids entre les monomères de 35:65 à 55:45, les copolymères étant partiellement neutralisés à hauteur de 2 à 15 % en moles avec des acides dicarboxyliques en C₃-C₁₀.

2. Revêtements selon la revendication 1, dans lesquels les agents de revêtement sont appliqués en quantités d'application de 1 à 20 mg/cm² et les revêtements présentent à des quantités d'application de 4 mg/cm² une résistance à la libération de l'agent actif dans un milieu aqueux à un pH de 6,8 d'au moins 80 % après 30 minutes.

3. Revêtements selon la revendication 1 ou 2, dans lesquels la quantité d'application de l'agent de revêtement est de 2 à 15 mg/cm.

4. Revêtements selon l'une quelconque des revendications 1 à 3, dans lesquels avec une quantité d'application de l'agent de revêtement de 4 à 12 mg/cm².

5. Revêtements selon l'une quelconque des revendications 1 à 4, dans lesquels les copolymères sont partiellement neutralisés avec des acides dicarboxyliques en C₃-C₁₀ non ramifiés.

6. Revêtements selon l'une quelconque des revendications 1 à 5, dans lesquels l'acide dicarboxylique est l'acide fumarique.

7. Revêtements selon l'une quelconque des revendications 1 à 6, dans lesquels les acides dicarboxyliques sont des acides alcanedicarboxyliques.

8. Revêtements selon l'une quelconque des revendications 1 à 7, dans lesquels les acides dicarboxyliques sont choisis dans le groupe constitué par l'acide malonique, l'acide succinique, l'acide glutarique, l'acide adipique ou l'acide sébacique, l'acide malique (acide 2-hydroxysuccinique) et l'acide tartrique (acide 2,3-dihydroxysuccinique).

9. Revêtements selon l'une quelconque des revendications 1 à 8, dans lesquels les copolymères sont partiellement neutralisés à hauteur de 4 à 10 % en moles.

10. Revêtements selon l'une quelconque des revendications 1 à 9, contenant des acides dicarboxyliques qui présentent une première valeur de pKₛ supérieure à 2 et une seconde valeur de pKₛ supérieure à 4.

11. Revêtements selon la revendication 10, contenant de l'acide adipique en tant qu'acide dicarboxylique.

12. Revêtements selon l'une quelconque des revendications 1 à 11, obtenus par application d'un agent de revêtement aqueux, qui est fabriqué par redispersion de l'agent de revêtement à partir de la forme pulvérulente.

13. Procédé de fabrication d'agents de revêtement pour des revêtements de formes posologiques contenant au moins un agent actif pharmaceutique présentant une résistance à la libération prématurée de l'agent actif, les agents de revêtement contenant en tant que polymères des copolymères de méthacrylate de N,N-diéthylaminoéthyle et de méthacrylate de méthyle en un rapport en poids entre les monomères de 35:65 à 55:45, **caractérisé en ce que** les copolymères sont partiellement neutralisés à hauteur de 2 à 15 % en moles avec des acides dicarboxyliques en C₃-C₁₀ dans l'agent de revêtement.

14. Procédé selon la revendication 13, **caractérisé en ce que** les copolymères filmogènes sont partiellement neutralisés avec les acides dicarboxyliques dans une dispersion aqueuse.

15. Procédé selon la revendication 14, **caractérisé en ce que** les copolymères filmogènes sont partiellement neutralisés sous forme pulvérulente.

16. Procédé selon l'une quelconque des revendications 13 à 15, **caractérisé en ce que** les copolymères filmogènes sont partiellement neutralisés à hauteur de 4 à 10 % en moles.

17. Procédé selon l'une quelconque des revendications 13 à 16, **caractérisé en ce que** les copolymères sont partiellement neutralisés avec des acides dicarboxyliques en C₃-C₁₀ non ramifiés.

18. Procédé selon l'une quelconque des revendications 13 à 17, **caractérisé en ce que** des acides alcanedicarboxyliques sont en tant qu'acides dicarboxyliques.

19. Procédé selon l'une quelconque des revendications 13 à 18, **caractérisé en ce que** les acides dicarboxyliques sont choisis dans le groupe constitué par l'acide malonique, l'acide succinique, l'acide glutarique, l'acide adipique ou l'acide sébacique, l'acide malique (acide 2-hydroxysuccinique) et l'acide tartrique (acide 2,3-dihydroxysuccinique).

20. Procédé selon l'une quelconque des revendications 13 à 19, **caractérisé en ce que** l'acide adipique est utilisé en tant qu'acide dicarboxylique.

21. Procédé selon l'une quelconque des revendications 13 à 20, **caractérisé en ce que** l'acide fumarique est utilisé en tant qu'acide dicarboxylique.

22. Procédé selon l'une quelconque des revendications 14 à 23, **caractérisé en ce que** les agents de revêtement pulvérulents sont redispersés dans de l'eau avant l'application sur la forme posologique.

23. Utilisation d'agents de revêtement selon l'une quelconque des revendications 14 à 24 pour des formes posologiques pharmaceutiques à base de copolymères de méthacrylate de N,N-diéthylaminoéthyle et de méthacrylate de méthyle en un rapport en poids entre les monomères de 35:65 à 55:45, les copolymères étant partiellement neutralisés à hauteur de 2 à 15 % en moles avec des acides dicarboxyliques en C₃-C₁₀, pour la protection de formes posologiques pharmaceutiques contenant un agent actif d'une libération prématurée de l'agent actif dans un milieu aqueux à un pH de 6,8.
